# EUROPEAN PATENT SPECIFICATION

(11) **EP 2 170 382 B1**
(45) Date of publication and mention of the grant of the patent: **31.07.2013**
(21) Application number: 07746958.3
(22) Date of filing: 15.06.2007
(51) Int. Cl.: A61K 39/145, C12N 7/04

(54) **LIVE VACCINE COMPRISING AN ATTENUATED INFLUENZA VIRUS**
LEBENDIMPFSTOFF EINEN ATTENUIERTEN INFLUENZAVIRUS ENTHALTEND
VACCIN VIVANT CONTENANT UN VIRUS DE LA GRIPPE ATTÉNUÉ

(43) Date of publication of application: 07.04.2010
(73) Proprietor: Protheon Co., Ltd., Seoul 120-749 (KR)
(72) Inventor: SEONG, Baik Lin, Seoul 135-120 (KR); LEE, Kwang Hee, Goyang-si (KR); SEO, Sang-Uk, Seoul 120-180 (KR)
(74) Representative: Bassil, Nicholas Charles
(86) International application number: PCT/KR2007/002924
(87) International publication number: WO 2008/153236

(56) References cited:
- EP-A2- 0 279 563
- WO-A1-99/28445
- WO-A2-01/83794
- WO-A2-2005/116258
- US-A- 5 149 531
- LEE K H ET AL: "Characterization of live influenza vaccine donor strain derived from cold-adaptation of X-31 virus" VACCINE, ELSEVIER LTD, GB LNKD- DOI:10.1016/J.VACCINE.2005.10.051, vol. 24, no. 11, 10 March 2006 (2006-03-10), pages 1966-1974, XP025151873 ISSN: 0264-410X [retrieved on 2006-03-10]
- HA S H ET AL: "A multiplex RT-PCR method for screening of reassortant live influenza vaccine virus strains" JOURNAL OF VIROLOGICAL METHODS, ELSEVIER BV, NL LNKD- DOI:10.1016/J.JVIROMET.2005.12.011, vol. 134, no. 1-2, 1 June 2006 (2006-06-01), pages 154-163, XP025030132 ISSN: 0166-0934 [retrieved on 2006-06-01]
- DATABASE GenBank [Online] NCBI; 9 August 2006 (2006-08-09), Lee, K.-H. et al.: "Influenza A virus (A/X-31(H3N2)) polymerase basic protein 2 (PB2) gene, complete cds" XP002599730 retrieved from NCBI Database accession no. DQ874873
- DATABASE GenBank [Online] NCBI; 9 August 2006 (2006-08-09), Lee, K.-H. et al.: "Influenza A virus (A/X-31(H3N2)) polymerase basic protein 1 (PB1) gene, complete cds" XP002599731 retrieved from NCBI Database accession no. DQ874874
- DATABASE Genbank [Online] NCBI; 9 August 2006 (2006-08-09), Lee, K.-H. et al.: "Influenza A virus (A/X-31(H3N2)) polymerase acidic protein (PA) gene, complete cds" XP002599732 retrieved from ncbi Database accession no. DQ874875
- DATABASE Genbank [Online] NCBI; 9 August 2006 (2006-08-09), Lee, K.-H. et al.: "Influenza A virus (A/X-31(H3N2)) nucleocapsid protein (NP) gene, complete cds" XP002599733 Database accession no. DQ874877
- DATABASE Genbank [Online] NCBI; 9 August 2006 (2006-08-09), Lee, K.-H. et al.: "Influenza A virus (A/X-31(H3N2)) matrix protein 2 and matrix protein 1 (M) gene, complete cds" XP002599734 retrieved from NCBI Database accession no. DQ874879
- DATABASE Genbank [Online] NCBI; 9 August 2006 (2006-08-09), Lee, K.-H. et al.: "Influenza A virus (A/X-31(H3N2)) nonstructural protein 2 and nonstructural protein 1 (NS) gene, complete cds" XP002599735 retrieved from NCBI Database accession no. DQ874880
- SEO ET AL: "Immediate and broad-spectrum protection against heterologous and heterotypic lethal challenge in mice by live influenza vaccine" VACCINE, ELSEVIER LTD, GB LNKD- DOI:10.1016/J.VACCINE.2007.09.012, vol. 25, no. 47, 26 October 2007 (2007-10-26), pages 8067-8076, XP022317483 ISSN: 0264-410X
- BAEZ M ET AL: "Gene composition of high-yielding influenza vaccine strains obtained by recombination." THE JOURNAL OF INFECTIOUS DISEASES MAR 1980 LNKD- PUBMED:7365284, vol. 141, no. 3, March 1980 (1980-03), pages 362-365, XP008126560 ISSN: 0022-1899
- MURPHY BRIAN R ET AL: "Principles underlying the development and use of live attenuated cold-adapted influenza A and B virus vaccines." VIRAL IMMUNOLOGY 2002 LNKD- PUBMED:12081014, vol. 15, no. 2, 2002, pages 295-323, XP002599736 ISSN: 0882-8245
- DATABASE GENEBANK [Online] NCBI 09 August 2006 LEE, K.-H ET AL.: 'Influenza A virus (A/X-31(H3N2)) hemagglutinin (HA) gene, complete cds' Retrieved from NCBI Database accession no. DQ874876
- DATABASE GENEBANK [Online] NCBI 09 August 2006 LEE, K.-H. ET AL: 'Influenza A virus (A/X-31(H3N2)) neuraminidase (NA) gene, complete cds' Retrieved from NCBI Database accession no. DQ874878

### sequence Listing

<110> PROTHEON CO., Ltd
<120> An attenuated influenza virus and a live vaccine comprising the same
<130> P49017EP-K/NCB
<140> EP07746958.3
   <141> 2007-06-15
<160> 18
<170> KopatentIn 1.71
<210> 1
   <211> 2295
   <212> DNA
   <213> Influenza virus
<400> 1
   ttcaatatgg aaagaataaa agaactacga aatctaatgt cgcagtctcg cacccgcgag      60
   atactcacaa aaaccaccgt ggaccatatg gccataatca agaagtacac atcaggaaga     120
   caggagaaga acccagcact taggatgaaa tggatgatgg caatgaaata tccaattaca     180
   gcagacaaga ggataacgga aatgattcct gagagaaatg agcaaggaca aactttatgg     240
   agtaaaatga atgatgccgg atcagaccga gtgatggtat cacctctggc tgtgacatgg     300
   tggaatagga atggaccaat aacaaataca gttcagtatc caaaaatcta caaaacttat     360
   tttgaaagag tcgaaaggct aaagcatgga acctttggcc ctgtccattt tagaaaccaa     420
   gtcaaaatac gtcggagagt tgacataaat cctggtcatg cagatctcag tgccaaggag     480
   gcacaggatg taatcatgga agttgttttc cctaacgaag tgggagccag gatactaaca     540
   tcggaatcgc aactaacgat aaccaaagag aagaaagaag aactccagga ttgcaaaatt     600
   tctcctttga tggttgcata catgttggag agagaactgg tccgcaaaac gagattcctc     660
   ccagtggctg gtggaacaag cagtgtgtac attgaagtgt tgcatttgac tcaaggaaca     720
   tgctgggaac agatgtatac tccaggaggg gaagtgagga atgatgatgt tgatcaaagc     780
   ttgattattg ctgctaggaa catagtgaga agagctgcag tatcagcaga tccactagca     840
   tctttattgg agatgtgcca cagcacacag attggtggaa ttaggatggt agacatcctt     900
   aggcagaacc caacagaaga gcaagccgtg gatatatgca aggctgcaat gggactgaga     960
   attagctcat ccttcagttt tggtggattc acatttaaga gaacaagcgg atcatcagtc    1020
   aagagagagg aagaggtgct tacgggaaat cttcaaacat tgaagataag agtgcatgag    1080
   ggatatgaag agttcacaat ggttgggaga agagcaacag ccatactcag aaaagcaacc    1140
   aggagattga ttcagctgat agtgagtggg agagacgaac agtcgattgc cgaagcaata    1200
   attgtggcca tggtattttc acaagaggat tgtatgataa aagcagtcag aggtgatctg    1260
   aatttcgtca atagggcgaa tcagcgattg aatcctatgc atcaactttt aagacatttt    1320
   cagaaggatg cgaaagtgct tttcaaaaat tggggagttg aacctatcga caatgtgatg    1380
   ggaatgattg ggatattgcc cgacatgact ccaagcatcg agatgtcaat gagaggagtg    1440
   agaatcagca aaatgggtgt agatgagtac tccagcacgg agagggtagt ggtgagcatt 1500
   gaccgttttt tgagaatccg ggaccaacga ggaaatgtac tactgtctcc cgaggaggtc 1560
   agtgaaacac agggaacaga gaaactgaca ataacttact catcgtcaat gatgtgggag 1620
   attaatggtc ctgaatcagt gttggtcaat acctatcaat ggatcatcag aaactgggaa 1680
   actgttaaaa ttcagtggtc ccagaaccct acaatgctat acaataaaat ggaatttgaa 1740
   ccatttcagt ctttagtacc taaggccagt agaggccaat acagtgggtt tgtaagaact 1800
   ctgttccaac aaatgaggga tgtgcttggg acatttgata ccgcacagat aataaaactt 1860
   cttcccttcg cagccgctcc accaaagcaa agtagaatgc agttctcctc atttactgtg 1920
   aatgtgaggg gatcaggaat gagaatactt gtaaggggca attctcctgt attcaactat 1980
   aacaaggcca cgaagagact cacagttctc ggaaaggatg ctggcacttt aactgaagac 2040
   ccagatgaag gcacagctgg agtggagtcc gctgttctga ggggattcct cattctgggc 2100
   aaagaagaca agagatatgg gccagcacta agcatcaatg aactgagcaa ccttgcgaaa 2160
   ggagagaagg ctaatgtgct aattgggcaa ggagacgtgg tgttggtaat gaaacggaaa 2220
   cgggactcta gcatacttac tgacagccag acagcgacca aaagaattcg gatggccatc 2280
   aattagtgtt gaata 2295
<210> 2
   <211> 2303
   <212> DNA
   <213> Influenza virus
<400> 2
   ttgaatggat gtcaatccga ccttactttt cttaaaagtg ccagcacaaa atgctataag 60
   cacaactttc ccttatactg gagaccctcc ttacagccat gggacaggaa caggatacac 120
   catggatact gtcaacagga cacatcagta ctcagaaaag ggaagatgga caacaaacac 180
   cgaaactgga gcaccgcaac tcaacccgat tgatgggcca ctgccagaag acaatgaacc 240
   aagtggttat gcccaaacag attgtgtatt ggaggcgatg gctttccttg aggaatccca 300
   tcctggtatt tttgaaaact cgtgtattga aacgatggag gttgttcagc aaacacgagt 360
   agacaagctg acacaaggcc gacagaccta tgactggact ctaaatagaa accaacctgc 420
   tgcaacagca ttggccaaca caatagaagt gttcagatca aatggcctca cggccaatga 480
   gtctggaagg ctcatagact tccttaagga tgtaatggag tcaatgaaca aagaagaaat 540
   ggggatcaca actcattttc agagaaagag acgggtgaga gacaatatga ctaagaaaat 600
   gataacacag agaacaatgg gtaaaaagaa gcagagattg aacaaaagga gttatctaat 660
   tagagcattg accctgaaca caatgaccaa agatgctgag agagggaagc taaaacggag 720
   aacaattgca accccaggga tgcaaataag ggggtttgta tactttgttg agacactggc 780
   aaggagtata tgtgagaaac ttgaacaatc agggttgcca gttggaggca atgagaagaa 840
   agcaaagttg gcaaatgttg taaggaagat gatgaccaat tctcaggaca ccgaactttc 900
   tttcaccatc actggagata acaccaaatg gaacgaaaat cagaatcctc ggatgttttt 960
   ggccatgatc acatatatga ccagaaatca gcccgaatgg ttcagaaatg ttctaagtat    1020
   tgctccaata atgttctcaa acaaaatggc gagactggga aaagggtata tgtttgagag    1080
   caagagtatg aaacttagaa ctcaaatacc tgcagaaatg ctagcaagca tcgatttgaa    1140
   atatttcaat gattcaacaa gaaagaagat tgaaaaaatc cgaccgctct taatagaggg    1200
   gactgcatca ttgagccctg gaatgatgat gggcatgttc aatatgttga gcactgtatt    1260
   aggcgtctcc atcctgaacc ttggacaaaa gagatacacc aagactactt actggtggga    1320
   tggtcttcaa tcctctgacg attttgctct gattgtgaat gcacccaatc atgaagggat    1380
   tcaagccgga gtcgacaggt tttatcgaac ctgtaagcta cttggaatca atatgagcaa    1440
   gaaaaagtct tacataaaca gaacaggtac atttgaattc acaagttttt tctatcgtta    1500
   tgggtttgtt gccaatttca gcatggagct tcccagtttt ggggtgtctg ggatcaacga    1560
   gtcagcggac atgagtattg gagttactgt catcaaaaac aatatgataa acaatgatct    1620
   tggtccagca acagctcaaa tggcccttca gttgttcatc aaagattaca ggtacacgta    1680
   ccgatgccat agaggtgaca cacaaataca aacccgaaga tcatttgaaa taaagaaact    1740
   gtgggagcaa acccgttcca aagctggact gctggtctcc gacggaggcc caaatttata    1800
   caacattaga aatctccaca ttcctgaagt ctgcctaaaa tgggaattga tggatgagga    1860
   ttaccagggg cgtttatgca acccactgaa cccatttgtc agccataaag aaattgaatc    1920
   aatgaacaat gcagtgatga tgccagcaca tggtccagcc aaaaacatgg agtatgatgc    1980
   tgttgcaaca acacactcct ggatccccaa aagaaatcga tccatcttga atacaagtca    2040
   aagaggagta cttgaggatg aacaaatgta ccaaaggtgc tgcaatttat ttgaaaaatt    2100
   cttccccagc agttcataca gaagaccagt cgggatatcc agtatggtgg aggctatggt    2160
   ttctagagcc cgaattgatg cacggattga tttcgaatct ggaaggataa agaaagaaga    2220
   gttcactgag atcatgaaga tctgttccac cattgaagag ctcagacggc aaaaatagtg    2280
   aatttagctt gtccttcatg aaa                                            2303
<210> 3
   <211> 2196
   <212> DNA
   <213> Influenza virus
<400> 3
   ccaaaatgga agattttgtg cgacaatgct tcaatccgat gattgtcgag cttgcggaaa 60
   aaacaatgaa agagtatggg gaggacctga aaatcgaaac aaacaaattt gcagcaatat 120
   gcactcactt ggaagtatgc ttcatgtatt cagattttca cttcatcaat gagcaaggcg 180
   agtcaataat cgtagaactt ggtgatccaa atgcactttt gaagcacaga tttgaaataa 240
   tcgagggaag agatcgcaca atggcctgga cagtagtaaa cagtatttgc aacactacag 300
   gggctgagaa accaaagttt ctaccagatt tgtatgatta caaggagaat agattcatcg 360
   aaattggagt aacaaggaga gaagttcaca tatactatct ggaaaaggcc aataaaatta 420
   aatctgagaa aacacacatc cacattttct cgttcactgg ggaagaaatg gccacaaagg 480
   cagactacac tctcgatgaa gaaagcaggg ctaggatcaa aaccagacta ttcaccataa 540
   gacaagaaat ggccagcaga ggcctctggg attcctttcg tcagtccgag agaggagaag 600
   agacaattga agaaaggttt gaaatcacag gaacaatgcg caagcttgcc gaccaaagtc 660
   tcccgccgaa cttctccagc cttgaaaatt ttagagccta tgtggatgga ttcgaaccga 720
   acggctacat tgagggcaag ctgtctcaaa tgtccaaaga agtaaatgct agaattgaac 780
   cttttttgaa aacaacacca cgaccactta gacttccgaa tgggcctccc tgttctcagc 840
   ggtccaaatt cctgctgatg gatgccttaa aattaagcat tgaggaccca agtcatgaag 900
   gagagggaat accgctatat gatgcaatca aatgcatgag aacattcttt ggatggaagg 960
   aacccaatgt tgttaaacca cacgaaaagg gaataaatcc aaattatctt ctgtcatgga 1020
   agcaagtact ggcagaactg caggacattg agaatgagga gaaaattcca aagactaaaa 1080
   atatgaagaa aacaagtcag ctaaagtggg cacttggtga gaacatggca ccagaaaagg 1140
   tagactttga cgactgtaaa gatgtaggtg atttgaagca atatgatagt gatgaaccag 1200
   aattgaggtc acttgcaagt tggattcaga atgagtttaa caaggcatgc gaactgacag 1260
   attcaagctg gatagagctc gatgagattg gagaagatgt ggctccaatt gaacacattg 1320
   caagcatgag aaggaattat ttcacatcag aggtgtctca ctgcagagcc acagaataca 1380
   taatgaaggg agtgtacatc aatactgcct tgcttaatgc atcttgtgca gcaatggatg 1440
   atttccaatt aattccaatg ataagcaagt gtagaactaa ggagggaagg cgaaagacca 1500
   acttgtatgg tttcatcata aaaggaagat cccacttaag gaatgacacc gacgtggtaa 1560
   actttgtgag catggagttt tctctcactg acccaagact tgaaccacat aaatgggaga 1620
   agtactgtgt tcttgagata ggagatatgc ttataagaag tgccataggc caggtttcaa 1680
   ggcccatgtt cttgtatgtg agaacaaatg gaacctcaaa aattaaaatg aaatggggaa 1740
   tggagatgag gcgttgcctc ctccagtcac ttcaacaaat tgagagtatg attgaagctg 1800
   agtcctctgt caaagagaaa gacatgacca aagagttctt tgagaacaaa tcagaaacat 1860
   ggcccattgg agagtccccc aaaggagtgg aggaaagttc cattgggaag gtctgcagga 1920
   ctttattagc aaagtcggta ttcaacagct tgtatgcatc tccacaacta gaaggatttt 1980
   cagctgaatc aagaaaactg cttcttgtcg ttcaggctct tagggacaac ctggaacctg 2040
   ggacctttga tcttgggggg ctatatgaag caattgagga gtgcctgatt aatgatccct 2100
   gggttttgct taatgcttct tggttcaact ccttccttac acatgcattg agttagttgt 2160
   ggcagtgcta ctatttgcta tccatactgt ccaaaa 2196
<210> 4
   <211> 1532
   <212> DNA
   <213> Influenza virus
<400> 4
   gataatcact cactgagtga catcaaaatc atggcgtctc aaggcaccaa acgatcttac 60
   gaacagatgg agactgatgg aggacgccag aatgccactg aaatcagagc atccgtcgga 120
   aaaatgattg gtggaattgg acgattctac atccaaatgt gcaccgaact caaactcagt 180
   gattatgagg gacggttgat ccaaaacagc ttaacaatag agagaatggt gctctctgct 240
   tttgacgaaa ggagaaataa ataccttgaa gaacatccca gtgcggggaa agatcctaag 300
   aaaactggag gacctatata caggagagta aacggaaagt ggatgagaga actcatcctt 360
   tatgacaaag aagaaataag gcgaatctgg cgccaagcta ataatggtga cgatgcaatg 420
   gctggtctga ctcacatgat gatctggcat tccaatttga atgatgcaac ttatcagagg 480
   acaagagctc ttgttcgcac cggaatggat cccaggatgt gctctctgat gcaaggttca 540
   actctcccta ggaggtctgg agccgcaggt gctgcagtca aaggagttgg aacaatggtg 600
   atggaattgg tcagaatgat caaacgtggg atcaatgatc ggaacttctg gaggggtgag 660
   aatggacgaa aaacaagaat tgcttatgaa agaatgtgca acattctcaa agggaaattt 720
   caaactgctg cacaaaaagc aatgatggat caagtgagag agagccggaa cccagggaat 780
   gctgagttcg aagatctcac ttttctagca cggtctgcac tcatattgag agggtcggtt 840
   gctcacaagt cctgcctgcc tgcctgtgtg tatggacctg ccgtagccag tgggtacgac 900
   tttgaaaggg agggatactc tctagtcgga atagaccctt tcagactgct tcaaaacagc 960
   caagtgtaca gcctaatcag accaaatgag aatccagcac acaagagtca actggtgtgg 1020
   atggcatgcc attctgccgc atttgaagat ctaagagtat taagcttcat caaagggacg 1080
   aaggtgctcc caagagggaa gctttccact agaggagttc aaattgcttc caatgaaaat 1140
   atggagacta tggaatcaag tacacttgaa ctgagaagca ggtactgggc cataaggacc 1200
   agaagtggag gaaacaccaa tcaacagagg gcatctgcgg gccaaatcag catacaacct 1260
   acgttctcag tacagagaaa tctccctttt gacagaacca ccattatggc agcattcaat 1320
   gggaatacag aggggagaac atctgacatg aggaccgaaa tcataaggat gatggaaagt 1380
   gcaagaccag aagatgtgtc tttccagggg cggggagtct tcgagctctc ggacgaaaag 1440
   gcagcgagcc cgatcgtgcc ttcctttgac atgagtaatg aaggatctta tttcttcgga 1500
   gacaatgcag aggagtacga caattaaaga aa 1532
<210> 5
   <211> 992
   <212> DNA
   <213> Influenza virus
<400> 5
   attgaaagat gagtcttcta accgaggtcg aaacgtacgt actctctatc atcccgtcag 60
   gccccctcaa agccgagatc gcacagagac ttgaagatgt ctttgcaggg aagaacaccg 120
   atcttgaggt tctcatggaa tggctaaaga caagaccaat cctgtcacct ctgactaagg 180
   ggattttagg atttgtgttc acgctcaccg tgcccagtga gcgaggactg cagcgtagac 240
   gctttgtcca aaatgccctt aatgggaacg gggatccaaa taacatggac aaagcagtta 300
   aactgtatag gaagctcaag agggagataa cattccatgg ggccaaagaa atctcactca 360
   gttattctgc tggtgcactt gccagttgta tgggcctcat atacaacagg atggggactg 420
   tgaccactga agtggcattt ggcctggtat gtgcaacctg tgaacagatt gctgactccc 480
   agcatcggtc tcataggcaa atggtgacaa caaccaatcc actaatcaga catgagaaca 540
   gaatggtttt agccagcact acagctaagg ctatggagca aatggctgga tcgagtgagc 600
   aagcagcaga ggccatggag gttgctagtc aggctagaca aatggtgcaa gcgatgagaa 660
   ccattgggac tcatcctagc tccagtgctg gtctgaaaaa tgatcttctt gaaaatttgc 720
   aggcctatca gaaacgaatg ggggtgcaga tgcaacggtt caagtgatcc tctcactatt 780
   gccgcaaata tcattgggat tttgcacttg acattgtgga ttcttgatcg tctttttttc 840
   aaatgcattt accgtcgctt taaatacgga ctgaaaggag ggccttctac ggaaggagtg 900
   ccaaagtcta tgagggaaga atatcgaaag gaacagcaga gtgctgtgga tgctgacgat 960
   ggtcattttg tcagcataga gctggagtaa aa 992
<210> 6
   <211> 852
   <212> DNA
   <213> Influenza virus
<400> 6
   aacataatgg atccaaacac tgtgtcaagc tttcaggtag attgctttct ttggcatgtc 60
   cgcaaacgag ttgcagacca agaactaggt gatgccccat tccttgatcg gcttcgccga 120
   gatcagaaat ccctaagagg aaggggcagt actctcggtc tggacatcaa gacagccaca 180
   cgtgctggaa agcagatagt ggagcggatt ctgaaagaag aatccgatga ggcacttaaa 240
   atgaccatgg cctctgtacc tgcgtcgcgt tacctaactg acatgactct tgaggaattg 300
   tcaagggact ggtccatgct catacccaag cagaaagtgg caggccctct ttgtatcaga 360
   atggaccagg cgatcatgga taagaacatc atactgaaag cgaacttcag tgtgattttt 420
   gaccggctgg agactctaat attgctaagg gctttcaccg aagagggagc aattgttggc 480
   gaaatttcac cattgccttc tcttccagga catactgctg aggatgtcaa aaatgcagtt 540
   ggagtcctca tcggaggact tgaatggaat gataacacag ttcgagtctc tgaaactcta 600
   cagagattcg cttggagaag cagtaatgag aatgggagac ctccactcac tccaaaacag 660
   aaacgagaaa tggcgggaac aattaggtca gaagtttgaa gaaataagat ggttgattga 720
   agaagtgaga cacaaactga agataacaga gaatagtttt gagcaaataa catttatgca 780
   agccttacat ctattgcttg aagtggagca agagataaga actttctcgt ttcagcttat 840
   ttagtactaa aa 852
<210> 7
   <211> 21
   <212> DNA
   <213> Influenza virus
<400> 7
   agcgaaagca ggtcaattat a 21
<210> 8
   <211> 18
   <212> DNA
   <213> Influenza virus
<400> 8
   agtagaaaca aggtcgtt 18
<210> 9
   <211> 20
   <212> DNA
   <213> Influenza virus
<400> 9
   agcgaaagca ggcaaaccat 20
<210> 10
   <211> 18
   <212> DNA
   <213> Influenza virus
<400> 10
   agtagaaaca aggcattt 18
<210> 11
   <211> 19
   <212> DNA
   <213> Influenza virus
<400> 11
   agcgaaagca ggtactgat 19
<210> 12
   <211> 18
   <212> DNA
   <213> Influenza virus
<400> 12
   agtagaaaca aggtactt 18
<210> 13
   <211> 15
   <212> DNA
   <213> Influenza virus
<400> 13
   agcaaaagca gggta 15
<210> 14
   <211> 18
   <212> DNA
   <213> Influenza virus
<400> 14
   agtagaaaca agggtatt 18
<210> 15
   <211> 17
   <212> DNA
   <213> Influenza virus
<400> 15
   agcgaaagca ggtagat 17
<210> 16
   <211> 18
   <212> DNA
   <213> Influenza virus
<400> 16
   agtagaaaca aggtagtt 18
<210> 17
   <211> 20
   <212> DNA
   <213> Influenza virus
<400> 17
   agcaaaagca gggtgacaaa 20
<210> 18
   <211> 18
   <212> DNA
   <213> Influenza virus
<400> 18 18
   agtagaaaca agggtgtt 18

## Description

### FIELD OF THE INVENTION

The present invention relates to a live vaccine comprising an attenuated influenza virus for use in early protection against a human influenza virus or an avian influenza virus.

### BACKGROUND OF THE INVENTION

Influenza A viruses are responsible for the major pandemics of influenza in the last century and are also the causative agents for most of the annual outbreaks of epidemic influenza. The influenza virus is an enveloped virus belonging to the family of the *Orlhomyxoviridae.* Within the envelope, the virus carries eight different RNA segments. There are many different influenza strains, and based on distinctive immunogenic properties, the virus are classified into three types: influenza A, B and C viruses (Influenza. Kilbourne ED ed. Plenum press 1987).

Influenza virus carries at least two different surface glycoprotein antigens on the external envelop, hemagglutinin (HA) trimer, consisting of three individual HA monomers, and the neuraminidase (NA) that exists as tetramer. Both HA and NA plays pivotal role during infection into susceptible cells. There are very immunogenic and elicits specific antibody responses during infection.

There are many different influenza A virus subtypes, differing in the nature of the HA and NA glycoproteins on their surface. Sixteen HAs (H1 to H16) and nine NAs (N1 to N9) have been identified. Among these, H1, H2 and H3 virus subtypes have been identified in humans, specifically the H1N1, H2N2 and H3N2 viruses corresponding to the three major pandemics of the last century (Fields Virology 4th ed. Fields BN, Knipe DM and Howly PM eds, 1489, 2001). Virus subtypes are distinguishable serologically, which means that antibodies to one subtype do not properly react with another subtype. Besides, humans, influenza viruses infect variety of hosts including swine, avian and equestrian species. Among these, aquatic birds appear to serve as a major reservoir of influenza A viruses; indeed, all human influenza virus subtypes circulate also in these wild birds. During resent outbreaks of avian influenza, there have been occasional transmissions of H5N1, H7N7 and H9N2 viruses to humans *(*Proc. Natl. Acad. Sci. USA 101, 8156-8161, 2004).

Besides influenza A subtypes, influenza B virus also infects humans especially very young children. Serologically, influenza B virus is clearly distinct from two different subtypes of influenza A viruses (A/H1N1 and A/H3N2) that currently circulates globally. For this reason, current influenza vaccine stipulates the use of three different components (A/H1N1, A/H3N2 and B virus) as trivalent vaccine formula.

Because of high mutation rate of the RNA genome, different strains emerge almost every year that cause annual influenza epidemics. Antigenic drift involves minor changes in the HA, NA and possibly also other viral antigen, that occur due to mutations in the viral genome, resulting in amino acid substitution in antigenic sites. These changes may render the new strain different enough to at least partially avoid the immunity induced by previous strains.

Since influenza virus genomes are segmented, influenza virus frequently undergoes more drastic changes 'antigenic shift' by genetic reassortnment between different viruses. This means that a virus with a new HA (and NA) are introduced into the human population. Especially among those who are immunologically naïve, the infection would spread rapidly and cause high morbidity and mortality among the entire population, including young healthy people. Human population has experienced at least three major influenza pandemics in the 20th century. The Spanish influenza (caused by an H1N1 influenza A virus) in 1918 resulted in the death toll of 20 to 50 million worldwide. The sequence of the virus shows that similar mutations were also observed in the recent H5N1 avian influenza isolates that infects humans (Nature 437, 889-93, 2005). Other pandemics occurred in 1957 (Asian flu, H2N2) and 1968 (Hong Kong flu, H3N2) with a total number of deaths of approximately 2 million. Moreover, there are numerous reports on human infection by avian influenza viruses (including H5N1, H7N7 and H9N2 viruses) in the past several years. The current information supports the concept that new pandemic influenza is derived from avian virus reservoirs. Avian influenza viruses may be directly transmitted to humans, which probably occurred in the case of the 1918 Spanish flu virus. The possibility of direct transmission of an avian influenza virus to humans became evident for the first time during the H5N1 outbreak in Hong Kong in 1997.

Recently, influenza B viruses have been isolated from animals, demonstrating that influenza B viruses are not restricted to humans and raising concerns about the potential for influenza B viruses to emerge with new antigenic properties. Moreover, influenza B virus infection has been associated with acute necrotizing encephalopathy (ANE) and influenza B-associated encephalitis (IBAE) and neurological sequela.

Vaccination remains the cornerstone of influenza prevention, and currently, trivalent vaccine containing three different influenza surface antigens (A/H1N1, A/H3N2 and B virus) are being used. Antigenic drift of established human virus subtypes requires regular update of the composition of the annual influenza vaccine necessitating annual vaccination. For this reason, current influenza vaccine stipulates the use of three different components as trivalent vaccine formula. In addition, because of potential circulation among humans of avian influenza viruses such as H5N1, H9N2 or H7N7, tetravalent and even multivalent vaccine can also be used for cross-protection against various influenza viruses.

There are several potential strategies for the development of vaccines to protect humans against influenza viruses, including (1) inactivated vaccine; (2) subunit vaccine that uses purified HA and NA components; and (3) live attenuated vaccines. Live attenuated vaccines generally provide better protection than inactivated on subunits vaccines as exemplified by previous developed A/Ann Arbor/6/60 (AA) (H2N2) virus or A/Lenhngrad/134/47/57 (H2N2) (Antiviral Res. 1, 339-365, 1981). The live vaccine stimulates the secretion of IgA class antibodies in the respiratory tract and inactivates the infecting virus on the infection site providing an on-site protection. (New Eng J Med. 338, 1405-1412, 1998; Vaccine 18, 82-88, 2000).

Classically, live attenuated vaccine has been developed through cold adaptation of infectious viruses (Antiviral Res. 1, 339-365, 1981). Using this virus as backbone strain, recombinant virus could be generated by annual reassortment between the ca virus and seasonal virulent influenza virus. The reassortant virus -carrying the surface antigens (HA ad NA) derived from the virulent strain and the six internal RNAs inherited from the ca virus- is immunologically identical to virulent strain but as attenuated as the parental ca virus. The virus, when given to human preferably through nasal route, does not cause virulent symptoms, and yet would induce specific protective immune response. Influenza vaccines are generally produced from virus grown on embryonated chicken eggs.

US 5,149,531 A discloses a cold-adapted reassortant live influenza virus for use as antiviral agent to treat a patient for influenza virus by immediate interference.

Lee et al (vaccine 24, 1966-1974, 2006) discloses a human influenza A virus X-31 (high-yielding strain) that was cold-adapted for possible future use as a live attenuated vaccine.

The Genbank accession numbers DQ874873, version 1, DQ874874, version 1, DQ874875, version 1, DQ874877, version 1, DQ874879, version 1, DQ874880, version 1 disclose the amino acid and nucleotide sequences of the influenza A virus (A/X-31 (H3N2)) polymerase basic protein 2, polymerase basic protein 1, polymerase acidic protein, nucleocapsid protein, matrix protein 2 and matrix protein 1, nonstructural protein 2 and nonstructural protein 1, respectively.

Ideally, a live vaccine should be both safe, effective in providing protection and cost-effective in production. For safety, the vaccine should be sufficiently attenuated and would not cause clinical symptom even at high dose of vaccination. For effective protection, the vaccine, even at low dose of vaccination, should be immunogenic enough to provide sufficient protection from virulent infection. Moreover, the vaccine strain must retain good growth property in embryonated eggs to reduce the production cost. The current global production capacity of egg-based influenza vaccine is very limited, with only 0.3 to 0.5 billion doses annually, which covers only 6-10 % of global population. In time of pandemics (H5N1 avian influenza, for example), the whole population is expected to suffer from the shortage of vaccine supply. Therefore, high-titre production of in embryonated eggs is especially important for influenza vaccine (WHO/CDS/CSR/RMD/2004.8).

In practice, however, it is usually difficult to generate a vaccine strain that satisfies all three requirements-safety, efficacy and the cost-effectiveness. This difficulty is inherently associated with the process of attenuation and the use of live vaccine. Usually, attenuation (providing safety) results in poor growth (at the expense of cost-effectiveness), and renders the virus less immunogenic (requiring high dose of vaccination). In addition, since there are many variants of influenza viruses, matching of surface antigens (HA and NA) between the vaccine and the virulent virus is really important for protection. Therefore, prediction of influenza strains should be made well ahead of actual circulation of seasonal influenza in winter time. If prediction fails, the vaccine of that particular influenza season is not effective. Unfortunately, the current inactivated influenza vaccine relies on specific, delayed immune response and does not provide enough cross-protection to different subtypes of influenza viruses (J Am Med Assoc 253, 1136-1139, 1985). Moreover, the vaccine does not provide protection from immediate infection (within one to five days before infection, for example), and should be given well ahead of infection, preferably at least 2-4 weeks before infection, to provide good protection. In contrast to seasonal influenza, the outbreak of avian influenza infection cannot be predicted (Cell 124: 665- 70, 2006). The current influenza vaccine therefore has intrinsic limitation in providing immediate protection against sudden influenza outbreaks with unknown identity.

As preparedness for newly emerging threat posed by avian influenza, WHO now recommends, in addition to stockpiling of antivirals, the development of pandemic vaccines suitable for mass immunization administered in non-invasive route (WHO/CDS/CSR/RMD/2004.8). Although vaccines and antiviral drugs constitute essential components for this purpose, they present intrinsic limitations as efficient control measures of pandemics: antivirals such as Tamiflu^{™} are usually effective only after infection whereas vaccines should be administered well ahead of infection, preferably at least 2 to 4 weeks before exposure to circulating viruses: Currently, there is no effective means for controlling immediate infection. This issue is important especially for avian influenza, where outbreak and the speed of spread among human population are unpredictable.

The limitation of the current influenza vaccine therefore calls for the development of novel vaccine and prophylactic strategies. Ideally, an efficient vaccine would provide both (1) broad-spectrum immediate prophylaxis against variety of influenza viruses of unknown identity, and (2) delayed but specific protection against circulating virus of which the identity has been predicted when used well ahead of infection, preferably 2-4 weeks before infection following generally recommended vaccination schedule.

Accordingly, the development of novel live influenza vaccine that addresses to all important issues: safety, efficacy, high production yield, immediate protection against variety of influenza subtypes and prolonged protection against specific influenza subtype is needed.

### SUMMARY OF THE INVENTION

The present invention provides a live vaccine comprising an attenuated influenza virus strain as an effective ingredient and at least one pharmaceutically acceptable carrier or excipient for use in early protection against a human influenza virus or an avian influenza virus, wherein the early protection is protection of the host from influenza infection at one day from vaccination, and wherein the attenuated influenza virus strain is preparable by cold adaptation of a mother strain which carries 6 internal genome segments of A/PR/8/34(H1N1) and two surface antigens hemagglutinin (HA) and neuramidase (NA) of A/Aichi/2/68(H3N2) and the attenuated influenza virus strain comprises RNA genome segments having nucleotide sequences corresponding to the DNA sequences of SEQ ID NOs: 1 to 6.

The PT-IV-01 based vaccine is useful for prevention of seasonal influenza episodes and sudden outbreak of influenza pandemics of predicted or unknown identity.

### BRIEF DESCRIPTION OF DRAWING

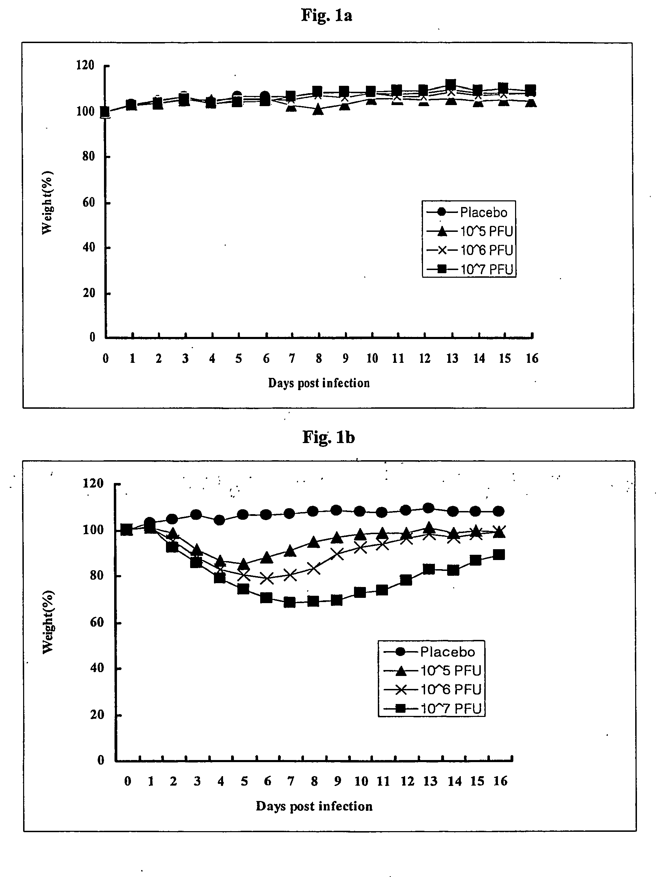

**Fig. 1a** is a graph showing effects of viral inoculation with PT-IV-01 on weight-loss in BALB/c mice. The PT-IV-01 virus was inoculated at different titers (10⁵-10⁷ PFU/nouse) and then weighted daily. PBS inoculated control (●), inoculated with 10⁵PFU/mouse (◢ ), inoculated with 10⁶ PFU/mouse (×) and inoculated with 10⁷ PFU/mouse (■).
**Fig. 1b** is a graph showing effects of viral inoculation with mother strain virus on weight-loss in BALB/c mice. Same symbols are used as fig. 1a for each experimental group.

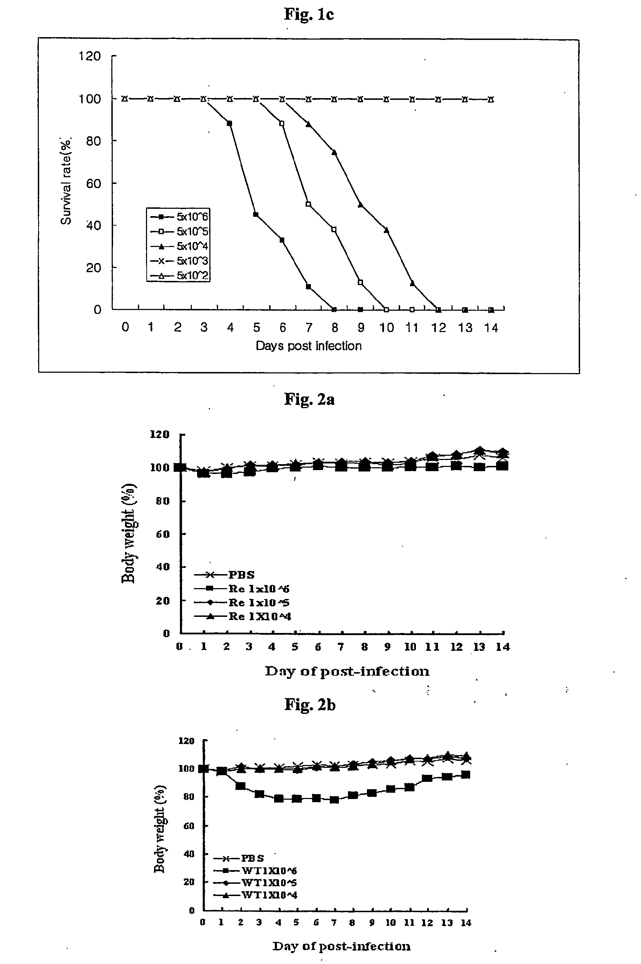

**Fig. 1c** is a graph showing effects of viral inoculation with A/PR/8/34 virus on survival rate in BALB/c mice. The A/PR/8/34 virus was inoculated at different titers (10²-10⁶ PFU/mouse) and the monitored daily. Inoculated with 10² PFU/mouse (Δ), inoculated with 10³ PFU/mouse (x), inoculated with 10⁴ PFU/mouse (◢ ), inoculated with 10⁵ PFU/mouse (□) and inoculated with 10⁶ PFU/mouse (■).
**Fig. 2a** is a graph showing body weight changes of BALB/c mice after intranasal infection with reassortant virus. Negative control (X), inoculated with 1X10⁶ PFU (■), (◆); inoculated with 1X10⁵ PFU (◆) and inoculated with 1X10⁴ PFU (◢ ).
**Fig.2b** is a graph showing body weight changes after intranasal infection with wild-type A/New Caledonia/20/99 (H1N1) viruses. Same symbols are used as fig. 2a for each experimental group.

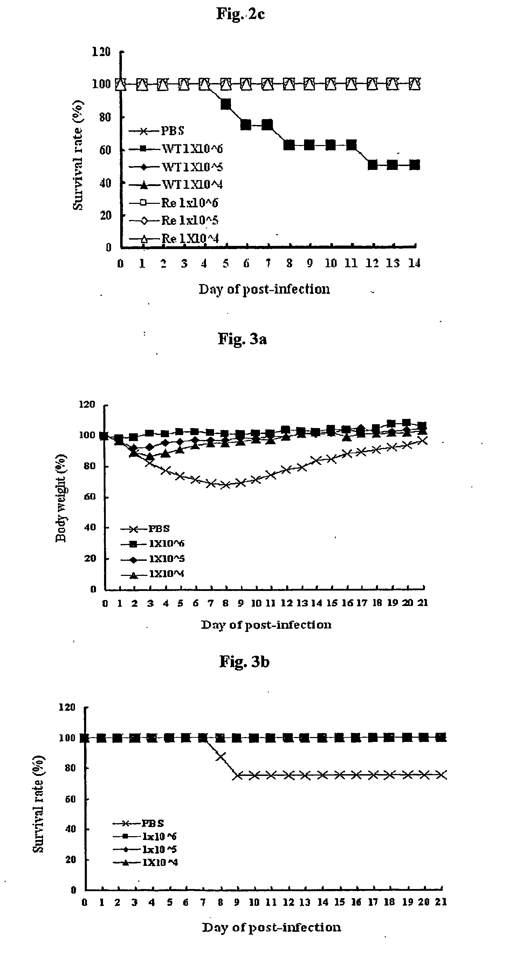

**Fig.2c** is a graph showing effects of viral inoculation with A/New Caledonia/20/99 (H1N1) reassortant viruses and wild-type viruses on survival rate in BALB/c mice. PBS control (X), inoculated with 1X10⁶ PFU of wild-type virus(■), inoculated with 1X10⁵ PFU of wild-type virus (◆) and inoculated with 1X10⁴ PFU of wild-type virus (◢ ), inoculated with 1X10⁶ PFU of reassortant virus (□), inoculated with 1X10⁵ PFU of reassortant virus (◊) and inoculated with 1X10⁴ PFU of reassortant virus (Δ).
**Fig.3a** is a graph showing body weight changes of six-week-old female BALB/c mice after challenge infection with A/New Caledonia/20/99 (H1N1) wild-type virus. PBS negative control (X), vaccinated with 1X10⁶ PFU of reassortant virus (■), vaccinated with 1X10⁵ PFU of reassortant virus (◆) and vaccinated with 1X10⁴ PFU of reassortant virus (◢).
**Fig.3b** is a graph showing survival rates after challenge infection with wild-type A/New Caledonia/20/99 (H1N1). Same symbols are used as fig. 3a for each experimental group.

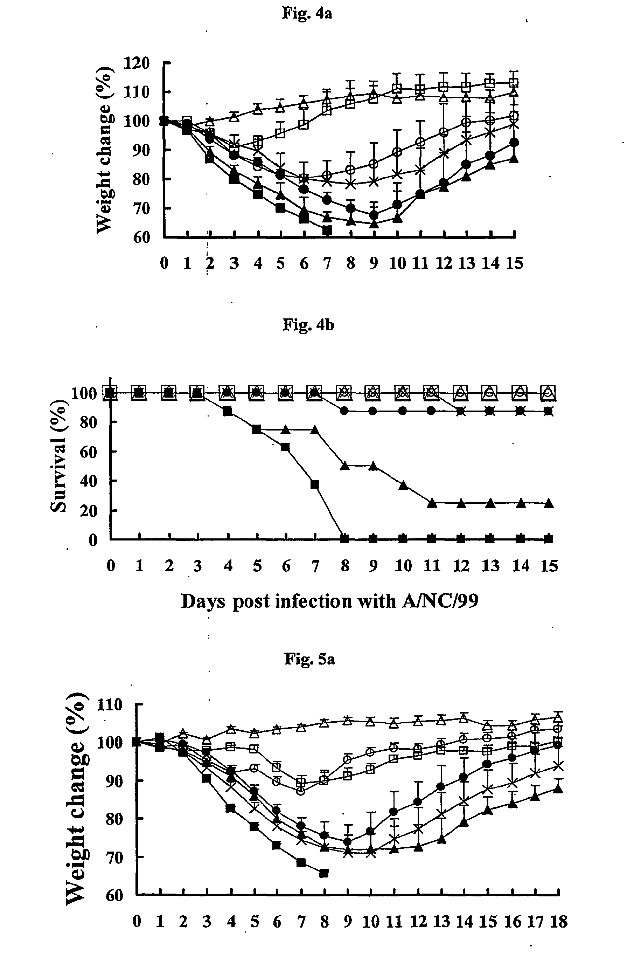

**Fig. 4a** is a graph showing body weight changes of BALB/c mice after challenge with lethal dose of A/New Caledonia/20/99 virus. PBS control (Δ), -4d group (□), -3d group (○), -2d group (x), -1d group (●), mix group (◢ ), without vaccination (■). The does for vaccination was 1.0×10⁶ PFU/mouse and the challenge with 2LD₅₀ (5.0×10⁵ PFU/mouse). Data are shown as means ± SEM (*n*=8).
**Fig. 4b** is a graph showing survival rate of mice in fig. 4a after challenge with A/New Caledonia/20/99. Same symbols are used as figure 4a for each experimental group.
**Fig. 5a** is a graph showing body weight changes of BALB/c mice after heterotypic protection provided by vaccination with PT-IV-01. Vaccination and challenge schedules are identical to experiment in figure 4a, except B/Shangdong/7/97 virus were used as challenge virus. PBS control (Δ), -4d group (□), -3d group (○), -2d group (x), -1d group (●), mix group (◢ ), without vaccination (■). The does for vaccination was 1.0×10⁶ PFU/nouse and the challenge with 2LD₅₀ (3.0×10⁵ PFU/mouse). Data are shown as means ± SEM (n=8).

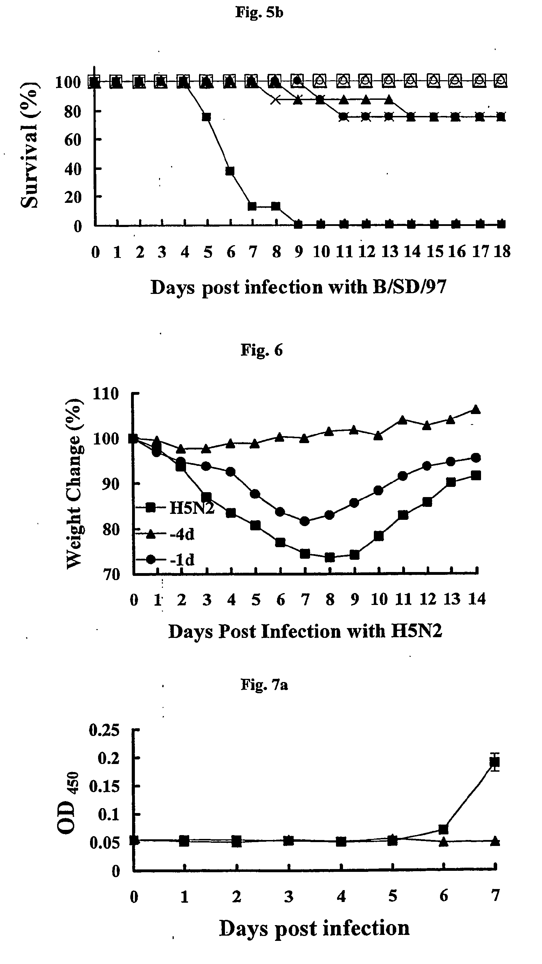

**Fig. 5b** is a graph showing urvival rate of mice in fig. 5a after challenge with B/Shangdong/7/97. Same symbols are used as figure 5a for each experimental group.
**Fig.6** is a graph showing body weight changes of BALB/c mice after vaccination with 1.0×10⁶ PFU of PT-IV-01 virus 1 (●) or 4 (◢ ) days before challenge. Mice were examined for early protection form avian influenza (H5N2) infection. Body weight was checked for 2 weeks. Results are compared to control group without vaccination (■).
**Fig. 7a** is a graph showing result of ELISA analysis for PT-IV-01 specific IgA in BAL fluid after vaccination with PT-IV-01. Mice were vaccinated (1.0×10⁶ PFU/mouse) to examine production of PT-IV-01 specific antibodies in vaccinated mice. PBS controlled (◢ ) and vaccinated (■) mice were analyzed daily for production of PT-IV-01 specific IgA in BAL fluid. Significant antibody level was detected 6 days post infection.

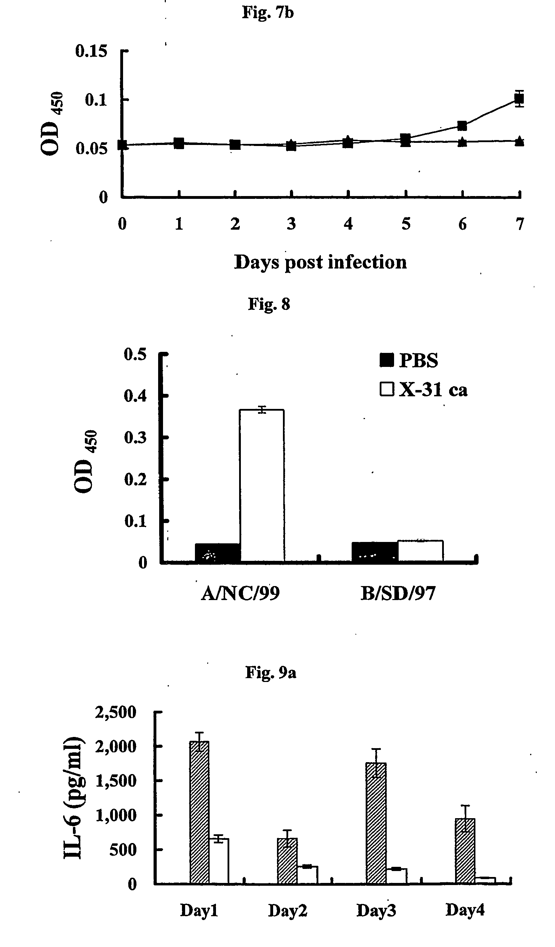

**Fig. 7b** is a graph showing results of ELISa anlysis for PT-IV-01 specific IgG in BAL fluid after vaccination with PT-IV-10. Serum samples were taken from same mice in figure 7a to examine PT-IV-01 specific IgG antibody production. Significant antibody level was detected 5 days post infection.
**Fig.8** is a graph showing cross-reactivity of serum IgG from mock (■) or PT-IV-01 (■) infected mice to A/NC199, and B/SD/97. Serums from PT-IV-01 vaccinated mice showed cross-reactivity to other heterologous influenza A virus (A/NC/99) but did not recognized heterotypic B/SD/97 virus. Data are shown as means ± SEM (n=8).

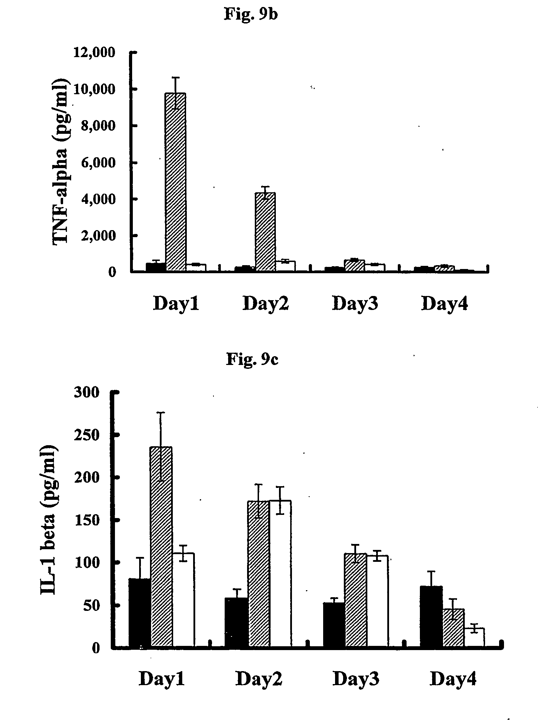

**Fig. 9** are graphs showing production of pro-inflammatory cytokines after vaccination with PT-IV-01 induces production of pro-inflammatory cytokines. The mean concentration of IL-6, TNF-α and IL-1β in BAL fluid were determined from mouse infected with mock (■), mother strain ( 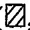 , 1.0×10⁶ PFU), and PT-IV-01 (□, 1.0×10⁶ PFU). Data are shown as means ± SEM and represents three independent experiments (n=12). ND; not detected.

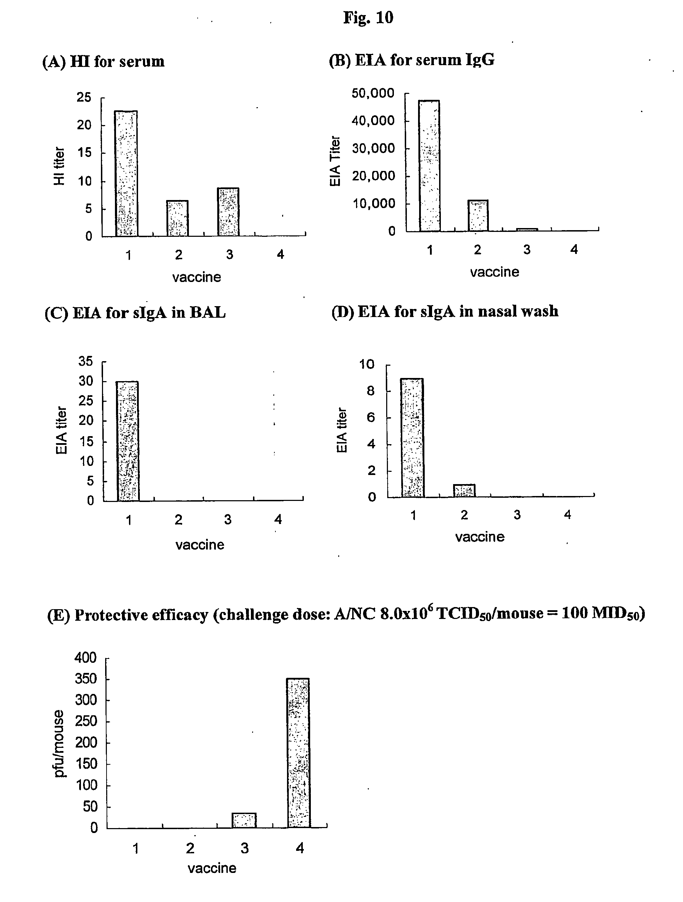

**Fig. 10** are graphs showing immunogenicity and protective efficacy of PT-IV-01 re(A/NC). PT-IV-01 re(A/NC) and mock were given by intranasal route, and the inactivated vaccine was injected by subcutaneous route. Hemagglutination inhibition (HI) for serum (a), enzymeimmuno assay (EIA) for serum IgG (b), EIA for slgA in BAL (c), EIA for slgA in nasal wash (d) and protective efficacy of vaccines (e) were performed. 1, PT-IV-01 re(A/NC). (8.9×10⁴ PFU); 2, PT-IV-01 re(A/NC) (8.9×10² PFU); 3, 3 x 0.75 ug of HA of inactivated trivalent (A/NC, A/PA, B/SD) split vaccine; 4, Mock.

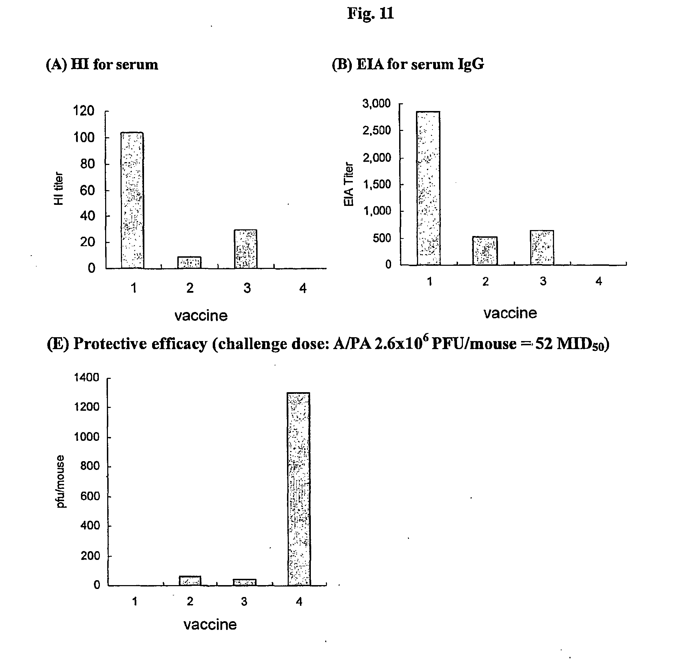

**Fig. 11** are graphs showing immunogenicity and protective efficacy of PT-IV-01 re(A/PA). Reassortant viruses and mock were given by intranasal route, but inactivated vaccine was injected by subcutaneous route. Hemagglutination inhibition (HI) for serum (a), enzymeimmuno assay (EIA) for serum IgG (b) and protective efficacy of vaccines (c) were performed. 1, PT-IV-01 re(A/PA). (1.9x10⁵ PFU); 2, PT-IV-01 re(A/PA) (1.9x10³ PFU); 3, 3 x 0.75 ug of HA of inactivated trivalent (A/NC, A/PA, B/SD) split vaccine; 4, Mock. (challenge dose ofA/Panama/2007/99: 2.6x10⁶ PFU/mouse = 52 MID₅₀)

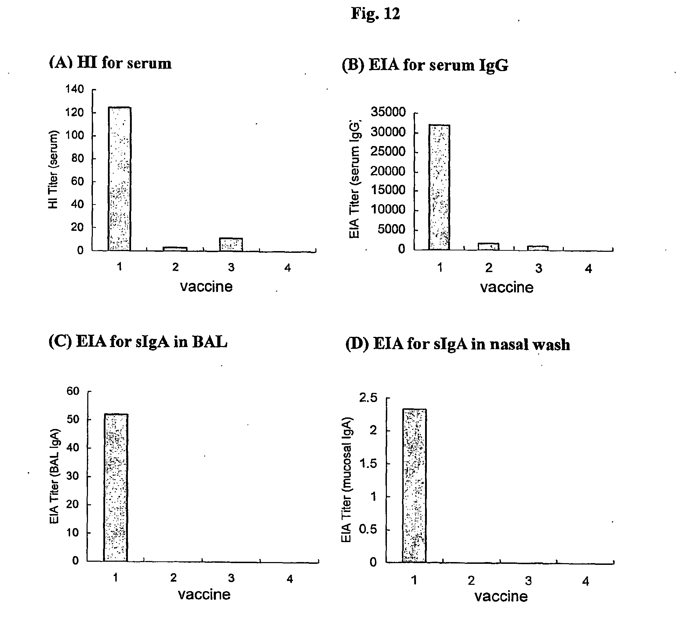

**Fig. 12** are graphs showing immunogenicity of of PT-IV-01 re(A/PA). Mice were vaccinated by intranasal route, and the inactivated vaccine was injected by subcutaneous route. Hemagglutination inhibition (HI) for serum (a), enzymeimmuno assay (EIA) for serum IgG (b), EIA for slgA in BAL (c) and protective efficacy of vaccines (d) were performed. 1, 1 x 10^{5.5} TCID₅₀ of PA-76-16; 2, 1 x 10^{3.5} TCID₅₀ of PA-76-16; 3, 3 x 7.5 ug of HA of inactivated trivalent (A/NC, A/PA, B/SD) split vaccine; 4, Mock.

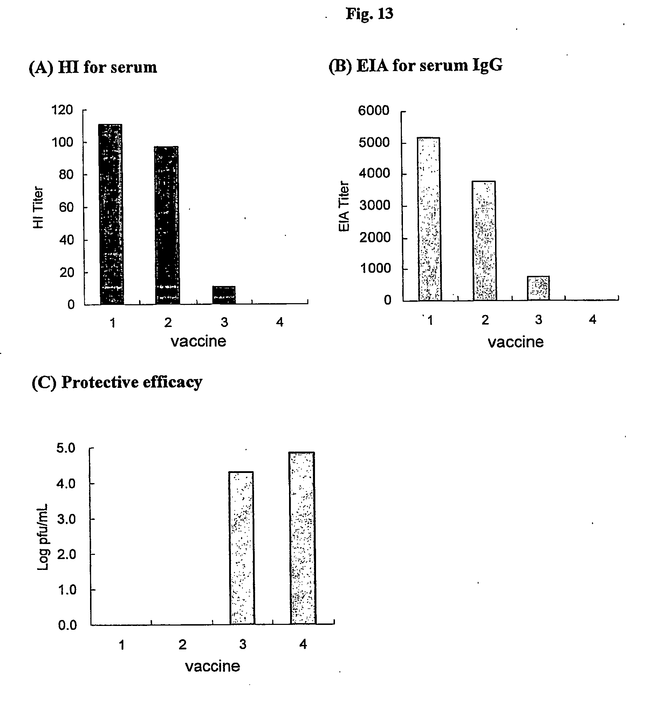

**Fig. 13** are graphs showing immunogenicity and protective efficacy of monovalent PT-IV-01 re(A/NC)(H1N1) or trivalent (PT-IV-01 re(A/NC)(H1N1), PT-IV-01 re(A/PA) (H3N2) and B/SD reassortant) vaccine. Reassortant vaccines and mock were given by intranasal route, but inactivated vaccine was injected by subcutaneous route. Hemagglutination inhibition (HI) for serum (a), enzymeimmuno assay (EIA) for serum IgG (b) and protective efficacy of vaccines (c) were performed. 1, PT-IV-01 re(A/NC) (1x10^{5.5} TCID₅₀); 2,·1x10^{5.5} TCID₅₀ each of trivalent vaccine (PT-IV-01 re(A/NC)(HIN1), PT-IV-01 re(A/PA) (H3N2) and B/SD reassortant); 3, 3 x 7.5 ug of HA of inactivated trivalent (A/NC, A/PA, B/SD) split vaccine; 4, Mock.

### DETAILED DESCRIPTION OF THE INVENTION

In a preferred embodiment, the human influenza virus is human influenza virus A or B.

The PT-IV-01 based vaccine is therefore useful for prevention of seasonal influenza episodes and sudden outbreak of influenza pandemics of predicted or unknown identity.

The present inventors initially pursued developing high-yielding live influenza virus vaccine donor strain. To this end, the present inventors began to passage a high-yielding influenza strain, a reassortant between A/PR/8/34(H1N1) and A/Aichi/2/68(H3N2) (hereafter, 'mother strain'). This virus carries 6 internal genomes of A/PR/8/34(H1N1) and two surface antigens HA and NA of A/Aichi/2/68(H3N2), and is therefore essentially identical to A/PR/8/34 in internal gene structure. Capitalizing on its high-growth property, the mother strain has long been used as a 'donor strain' for annual generation of reassortant viruses that carry 6 internal genes of A/PR/8/34 backbone and HA and NA genes from the epidemic strains of particular influenza season. During the process of isolating homogeneous population of vaccine viruses and characterization of their phenotypes, the present inventors came across a novel influenza isolate (PT-IN-01) that has virtually all the dsired attributes of an ideal vaccine as described above. Moreover, using PT-IN-01 strain, reassortant viruses, PT-IN-01 re, could be generated that carry the HA and NA surface antigens from current virulent viruses and the 6 internal RNA genomes of A/PR/8/34 origin inherited from the cold-adapted PT-IN-01. The PT-IN-01 re as attenuated as the parental PT-IV-01 strain but immunologically identical to the virulent virus. The PT-IN-01 re therefore is suitable for conferring long-term protection when given well ahead of infection by virulent viruses.

The vaccine not only provides the usual antibody-mediated protection when given sufficient time ahead of infection, but provides extremely fast protection within 1-4 days before infection even in the absence of specific antibody responses. Surprisingly, the vaccine conferred broad-spectrum protection against various subtypes of influenza viruses including human influenza A/H1N1, A/H3N2 viruses and influenza B virus and avian influenza virus as well. For this purpose, either the parental PT-IV-01 or the reassortant PT-IV-01 re could be used since early protection does not depend on the nature of surface antigens. Therefore, the vaccine is ideal for pre-planned protection against the seasonal human influenza and immediate protection from sudden influenza outbreaks including avian influenza pandemics. A broad-spectrum and immediate protection provided by PT-IV-01 or the reassortant PT-IV-01 re may offer a prompt measure for minimizing initial spread of virus and mitigating the impact of unexpected influenza outbreaks.

Unlike previously developed live influenza vaccine strain that provides partial therapeutic effects against sub-lethal dose of challenge , the PT-IV-01 exhibits novel effects hitherto unknown: 1) protection even against lethal challenge, 2) extremely broad protection against different subtypes including H5 type avian influenza virus, 3) broad-spectrum protection against lethal challenge of influenza A (heterologous) or B virus (heterotypic). and 4) better protection against more virulent viruses.

The novel immediate and broad spectrum protection was achieved in the absence of specific antibody response. Vaccination immediately prior to challenge resulted in the generation of significant pool of reassortant viruses between the vaccine and virulent strains suggesting that the acquisition of attenuating gene(s) from the live vaccine contributes in part to the attenuation of virulence. More importantly, the vaccination also resulted in immediate release of marker pro-inflammatory cytokines for innate immune response, such as IL-6, IL-1β and TNF-α (Nat Med 11, s23-28, 2005). The results suggest that innate immune responses play an important role in immediate protection (Science 300: 1524-25, 2003). It should be noted that vaccine efficacy could be increased by implementing with innate immune boosting effect (Nat Med 11: S63-68, 2005). Thus, genetic interference and innate immune responses play a role independently and synergistically towards early protection by live influenza vaccination. However, a poor (or rather inverse) correlation between the genetic interference and protection efficacy suggests that actually the innate immune response plays much greater role in early protection. Moreover, when given well ahead of infection (2-4 weeks before infection), the vaccine stimulated specific antibody response conferring extremely efficient long-term protection against virulent infection. Based on these hitherto unknown novel properties, PT-IV-01 is suitable for dual use purpose; for both immediate broad-spectrum protection against unknown influenza species of which the identity is yet to be confirmed, and long-term prolonged protection against predicted or circulating influenza subtypes.

Ideal attributes for live vaccine comprises safety, efficacy, user-friendliness and high productivity. Usually, safety of live vaccine is correlated with reduced growth and infectivity compromising the efficacy and the productivity of vaccine. To overcome this hurdle, a judicious choice of maternal strain from which vaccine strain is derived from is important. Here, the vaccine strain used for preparation of the attenuated influenza virus strain used in the present invention is derived from high-yielding influenza strain (a reassortant between A/HK/6/68 and A/PR/8/34; hereafter 'mother strain'). This mother strain carries 6 internal genomes of A/PR/8/34(H1N1) and two surface antigens HA and NA of A/Aichi/2/68(H3N2). As a backbone strain for the production of killed or inactivated influenza vaccine, the mother strain has been passaged in embryonated eggs for many years, and therefore retained high-growth phenotype in eggs and host-range attenuated phenotypes (J infect Dis 141, 362-365, 1980). Here the mother strain was subjected to repeated passage at low temperature (cold-adaptation) for further attenuation.. This would render the vaccine strain extremely safe since the strain underwent attenuation procedure twice and attained double layer of safety, and yet the vaccine strain retains high growth phenotype as the mother strain. After extensive search from the pool of the cold-adopted virus, we came across a novel vaccine strain PT-IV-01 that provides hitherto unknown property of extremely early protection against variety of influenza strains and subtypes. PL-TV-01 could be administered in a non-invasive manner preferably through nasal route.

The novel attenuated influenza virus strain and reassortant influenza virus strain used in the present invention was prepared as follows briefly:

### Generation of attenuated influenza virus by repeated passage at low temperature

Specific pathogen free (SPF) eggs were used for continuous passage of the mother strain for cold-adaptation. Fertilized eggs were incubated at 37C for 10 to 11day for embryogenesis. The embryonated eggs were briefly incubated at desired temperatures for 3-5 hours before virus inoculation. Each group of embryonated eggs (5 eggs/group) was inoculated via allantoic route with 100u1 of virus solution containing 0.1 to 0.001 hemagglutination units (HAU). Allantoic fluids were harvested after 3 to 4 days of infection and the virus titer was estimates by hemagglutination (HA) assay. The allantoic fluid that exhibited highest virus titer was selected and used for the subsequent passage. Initial passage was conducted at 30°C for 19 times. Then subsequent passage was followed: 40 times at 27°C and finally 33 times at 24°C.

### Isolation of PT-IV-01 strain

Madin-Darby canine kidney (MDCK) cells were grown in minimal essential medium (MEM) supplemented with 10% fetal bovine serum (FBS). The cells were cultured as monolayers in the MEM medium supplemented with 10% heat-inactivated fetal bovine serum. The cells were used for plaque purification of the virus that underwent final passage at 24 °C. Confluent MDCK cells were washed with serum free MEM media and incubated at room temperature with ten fold dilutions in MEM of allantoic fluid of 24°C passaged eggs. The inoculum was then removed, and the cells were overlaid with medium containing 10µg/ml trypsin and 1% low-melting agarose and incubated at 37°C in 5% CO₂ incubator. Individual plaques were isolated, and repeated plaque assay was performed to ensure the genetic homogeneity of the isolate. Each plaque was then amplified in embryonated eggs and examined for various characters and parameters indicative of safety, efficacy and productivity. One independent isolate that carried all desirable characters was named as PT-IV-01 and deposited on April 24, 2007 at KCCM (Korea Culture Center of Microorganisms) located in #361-221, Yurim B/D, Hongje-1-dong, Seodaemun-gu, SEOUL 120-091, Republic of Korea under Accession NO: KCCM 10854P in compliance with the Budapest Treaty on the International Recognition of the Deposit of Microorganisms for the Purpose of Patent Procedure. The depositor of the microorganism assures that (a) all restrictions on the availability to the public of the deposited material referred to in paragraph 2 will be irrevocably removed upon issuance of a United States patent of which any of such deposited material is the subject; (b) the deposited material will be maintained for a period of at least five years after the most recent request for the furnishing of a sample of any of the deposited material was received by the KCTC and, in any case, for a period of at least 30 years after the date of deposit or for the effective life of such patent, whichever is longer; (c) should any of the deposits become non-viable or mutated, or otherwise incapable of being furnished by the depository upon request due to the condition of the deposit, it will be replaced by Applicants; and (d) access to the cultures will be available to the Commissioner during the pendency of the patent application or to one determined by the Commissioner to be entitled to such cultures under 37 C.F.R. §1.14 and 35 U.S.C. §122.

The RNAs were isolated from the virus, and using the segment specific primers, RT-PCR was performed and the sequence was verified for six internal RNA genomes (PB2, PB1, PA, NP, M, NS, respectively) as provided in the sequence listing (SEQ ID NOs: 1 - 6).

### Characterization of ts, ca, att phenotypes of PT-IV-01

The temperature sensitive*(ts)*, cold-adapted *(ca)* and attenuated *(att)* phenotypes of PT-IV-01 were evaluated *in vitro, in ovo and in vivo.* The growth characteristics of PT-IV-01 were evaluated at different temperature in ovo as follows. Embryonated eggs were inoculated with 0.1 HAU of PT-IV-01 or reassortants therof (PT-IV-01 re) and incubated at 25°C, 30°C and 37°C. Allantoic fluids were harvested 3 days after inoculation and the yield of infectious virus was assayed at 30°C in MDCK cells. Likewise, the growth *in vitro* was evaluated by infection and culture in MDCK cells at different temperatures.

The attenuation phenotype of PT-IV-01 was evaluated by nasal administration of mice. For each experiment, a group of eight mice were immunized intranasally with the vaccine strain. Six-week-old female BALB/c mice were housed in filter-isolate cages. The mice were inoculated 5 days after arrival. Prior to inoculation (day 0), the mice were anesthetized with ketamine (0.4 mg/mice). Fifty microliters of virus inoculum were delivered intra-nasally by 200ul pipet tip to the anesthetized mice. The body weight and any clinical signs were monitored daily.

### Generation and identification of reassortant viruses

Reassortant virus was generated by the following procedure. 11-day-old embryonated eggs were co-infected with the cold-adapted PT-IV-01 and wild-type virus at a same titer and incubated at 30°C. After 3 days incubation, allantoic fluids were harvested. To select 6 : 2 reassonant virus from these random reassortant mixture, viruses were passaged in the presence of antiserum against PT-IV-01 at 25 °C in MDCK or primary chicken kidney (PCK) cells. The viruses were selected by plaque assay in the presence of anti PT-IV-01 antiserum at 25°C. Here, the successful selection of desired reaasortant viruses among two different viruses usually depends on the quality of the antiserum against the donor virus.

The antiserum for PT-IV-01 virus was generated by the following procedure. The virus PT-IV-01 were cultured in eggs and purified by ultracentifugation. Viruses are adsorbed to adjuvant such as Al(OH)₃ and are injected into guinea pigs and rabbits via subcutaneous route. After one month, the serum was taken from the immunized animals, separated by centrifugation and inactivated by treating at high temperature. And then the serum is treated with kaolin to reduce the non-specific reaction between RBC (red blood cells) and serum. The serum is then cleared of small debris by passing through 0.22 um filter, and it is then stored in small aliquot at -70 °C until use. The reassortant viruses that carry surface antigens of virulent origin would not be neutralized by the antiserum and therefore survive the treatment and make plaques in this process.

The plaque purification can be done using the MDCK cells or primary chicken kidney (PCK) cells.The PCK cells were obtained by the following procedure. The 18∼19-day-old embryonated hen's eggs were sanitized with 70% ethanol and opened by scissors. Embryo was withdrawn from the egg, and the legs were removed to help isolation of kidneys located near backbone. The kidneys were washed with PBS, and then the connective or fat tissues attached to the kidney were removed carefully. The kidneys were washed again with PBS and transferred to a small beaker. The kidneys were chopped by scissors and washed with PBS, and then pre-heated PBS and trypsin (usually 2.5%(w/v)) were added. After incubation with gentle stirring, the cell suspension was passed through gauge filter on the funnel/flask containing FBS. The cells were then transferred to a new tube and washed with growth medium. After the propagation of the cells in T-flasks, the cells were harvested and preserved at liquid nitrogen gas tank until use.

Isolated plaques from PCK or MDCK cells were then amplified in embryonated chicken eggs. The ca and *ts* phenotype of plaque purified viruses were determined according to their plaquing efficiency at different temperatures in the range of 25°C to 40°C in MDCK or PCK cells. Finally, the identities of their genetic components were analyzed by RT-PCR dependent genome analysis method.

For characterization of the reassortant viruses, it is very important to finally identify and confirm the origin of eight RNA genomes. For this purpose, the RNA genes could be reverse transcrided into cDNA by RT-PCR (reverse transcription - polymerase chain reaction) and the sequence of the cDNA can be determied. Generally, RNAs isolated from virus is initially reverse transcribed with universal primer. And then, selective RNA segment could be further amplified by the segment-specific primers. For this purpose, segment specific primers could be designed for selective amplification of particular RNA genome. If the size of the amplified DNAs derived from various RNA segments is sufficiently different, then the specific primer sets can be combined and more than two different RNAs can be amplified in a multiplex manner. After final identification of desired 6:2 RNA ratio, then the nuclotide sequences of genome segments for HA and NA are finally determined. This is a final check for the genetic stability of HA and NA during reassortment process. Since there always is a possibility of nucleotide change in the RNA genome due to high mutation rate intrinsic to infleunza virus, the comparison of the sequences between before and after reassortment process as a final check is important.

### Immunization, broad-spectrum immediate prophylaxis and long-term protection

The prophylactic effect of vaccine could be evaluated by experimental infection of susceptible animals (5-6 week old BALB/c mice, for example). To ensure statistical significance for the results, eight animals per experimental group are used. For immediate protection, mice were vaccinated daily by intranasal route with varying dose of PT-IV-01 or reassortant virus (PT-IV-01 re) thereof, up to four days prior to challenge. Mice were usually anesthetized before receiving lethal dose of virulent virus, such as A/New Caledonia/99 or B/Shangdong/97. In parallel, therapeutic effect of the vaccine virus can also be evaluated by coinfection of mice with virulent virus in time of vaccination. Here, the dose of both virulent and the vaccine viruses could be varied to evaluate the optimal condition for prophylaxis or therapeutic effect. In general, varying titers (1.0×10³ to 1.0×10⁶ PFU) of vaccine dose are used before lethal challenge with about 5-10 LD₅₀ of virulent viruses. Alternatively, vaccination was also performed according to standard procedures: a month interval between vaccination and challenge. All mouse studies were performed under the regulation of the Animal Care Committee at Yonsei University.

### Antibodies, proinflammatory cytokines and genetic interference during vaccination and challenge.

After vaccination of animals, the level of antibodies can be determined by enzyme-linked immunosorbent assay (ELISA). Therefore, in a certain time interval, each group of mice were sacrificed by cervical dislocation and bronchoalveolar lavage (BAL) fluid was for the titration of IgA and cytokine levels. IgG titration was performed using serum obtained from the same mice (twelve animals per experimental group). For the titration of IgG and IgA antibodies, a microwell plate coated with antigen, preferably the challenge virus A/NC/99 or B/SD/97, are usually used.

Induction of the antiviral innate immune response is mediated by host pattern-recognition receptors including Toll-like receptor (TLR), which provides first hand protection from various viral infection (Nat Immunol 7, 131-137, 2006). During this process, TLR family members sense various pathogens through pattern recognition receptors to activate common signaling pathways (J Pediatr 144, 421-429, 2004). To evaluate whether the vaccination elicit innate immune response, the marker cytokines for innate immune response, such as IL-6, TNF-α and IL-1β are monitored after vaccination. Alternatively, type 1 antiviral responses are monitored by measuring IFN-α.

Administration of the live vaccine disclosed above to an individual can be by any known or standard technique. These include oral ingestion, gastric intubation, or broncho-nasal-ocular spraying. All of these methods allow the live vaccine to easily reach the NALT, GALT or BALT cells and induce antibody formation and cell mediated immunity and are the preferred methods of administration. Other methods of administration, such as intravenous injection, that allow the carrier microbe to reach the individual's blood stream can be acceptable. Intravenous, intranasal, intramuscular or intramammary injection are also acceptable, as is described later.

The immunization dosages required will vary with the antigenicity of the live vaccine product and need only be an amount sufficient to induce an immune response. Routine experimentation will easily establish the required amount. Multiple dosages are used as needed to provide the desired level of protection.

The pharmaceutical acceptable carrier or excipient in which the live vaccine is suspended or dissolved may be any solvent or solid or encapsulating material such as for a lypholized form of the vaccine. The carrier is non-toxic to the inoculated individual and compatible with the microorganism or antigenic gene product. Suitable pharmaceutical carriers are known in the art and, for example, include liquid carriers, such as normal saline and other non-toxic salts at or near physiological concentrations, and solid carriers, such as talc or sucrose. Gelatin capsules can serve as carriers for lypholized vaccines. Adjuvants may be added to enhance the antigenicity if desired. When used for administering via the bronchial tubes, the vaccine is preferably presented in the form of an aerosol. Suitable pharmaceutical carriers and adjuvants and the preparation of dosage forms are described in, for example, Remington's Pharmaceutical Sciences, 17th Edition, (Gennaro, Ed., Mack Publishing Co., Easton, Pa., 1985).

The present invention will be described in more detail with examples. The following examples are only for demonstration of the present invention, but dose not limit the range of the claims of the present invention.

### <Example 1> Cold-adaptation of influenza A virus

The mother strain was subjected to repeated passage in embryonated chicken eggs at progressively lower temperatures to select virus with mutations that would produce a cold-adapted and temperature-sensitive phenotypes. Eggs were incubated at 30°C, 27°C or 24°C for 3-5 hours before virus inoculation and then incubated at the same temperature after inoculation. For each temperature, 5 or 6 embryonated eggs were injected via allantoic route. The inoculum (100µl) contained 0.001HAU to 0.1HAU. Infected allantoic fluids were harvested after 72 or 96 hours and titrated by HA assay. Among these, the highest growth virus were selected and used for the next passage. Following the procedure, the mother strain was serially passaged 19 times in embryonated chicken eggs at 30°C and then serially passaged additional 40 times at 27°C. Finally, this virus was further passaged 33 times in embryonated chicken eggs at 24°C. This cold-adapted virus was plaque purified three times and amplified at 24°C to make working stocks. An isolate was named PT-IV-01, and has been deposited at Korea Culture Center of Microorganisms, Korea, on April 24, 2007 under Accession number: KCCM 10854P.

### <Example 2> Growth characterization of PT-IV-01 cold-adapted influenza virus in embryonted chicken eggs

The growth characteristics of PT-IV-01 was examined and compared with the mother strain in embryonated chicken eggs. Embryonated eggs were inoculated with 0.1 HAU of PT-IV-01 or the mother strain and incubated at 37°C, 30°C and 25°C. Allantoic fluid was harvested 3 days after inoculation and titrated with HA assay. As shown in Table 1, PT-IV-01 virus grew in eggs at 25°C, whereby the mother strain was not detected in HA assay. When growth pattern of the PT-IV-01 and the mother strain were compared at 37°C, the yield of the PT-IV-01 virus was significantly lower than the mother strain. As a result, the cold-passaged PT-IV-01 virus exhibited both cold-adapted phenotype and restricted growth phenotype at body temperature in embryonated chicken eggs. Overall, the growth profile is shifted in favor of low temperature thanks to repeated passage at low temperature. Notably, the virus titer of the PT-IV-01 was comparable to that of the mother strain, suggesting that even after extensive attenuation procedure, high growth phenotype is still maintained.

**Table 1**

| strain | Vims titer (log2 HAU) | | |
|---|---|---|---|
| | 25°C | 30°C | 37°C₂₅ |
| PT-IV-01 | 7±0.5 | 11±1 | 6±1 |
| Mother strain | - | 11±1 | 11±1 |

### <Example 3> Growth characterization of PT-IV-01 cold-adapted influenza virus in animal cell line.

The ca and *ts* properties of the PT-IV-01 virus were assessed by plaque assay on MDCK cell at both low and high temperatures. Plaque forming unit (PFU) were compared at three different temperature, 25 °C, 33°C and 39°C. The plates of infected virus were incubated 5-7 days at 25°C and 2-3 days at 33°C and 39°C. Growth at 33 °C was used as the standard condition.

As shown in Table 2, the PT-IV-01 virus formed plaques efficiently at 25°C, but inefficiently at 39°C. The result showed that PT-IV-01 virus had appropriate cold-adapted and temperature-sensitive properties in animal cell lines, extending similar observations in embryonated eggs. Clearly, the optimal temperature for plaque formation was shifted in favor of low temperature. It is woth noting that the virus titer of PT-IV-01 (8.1-8.6 log10 pfu/ml) is similar to that of the mother strain (8.4-8.5 log10 pfu/ml), suggesting that high growth phenotype is still maintained. The results, suggests that MDCK certified cell lines could be used as substrates for the production of PT-IV-01 influenza vaccine in good yields.

**Table 2**

| Strain | Virus titer (log10 pfu/ml) | | | 5 |
|---|---|---|---|---|
| | 25°C | 33°C | 39°C | |
| PT-IV-01 | 8.1 | 8.6 | 2.8 | |
| Mother strain | 2.1 | 8.5 | 8.4 | |

### <Example 4> Characterization of PT-IV-01 cold-adapted influenza virus in mice.

Six-week-old female BALB/c mice were used as a laboratory model to assess potential virulence of the PT-IV-01 virus. BALB/c mice were infected intranasally with 50µl of the virus at different dilutions. After inoculation with varying dose of PT-IV-01 or the mother strain (10⁵-10⁷ PFU/mouse), clinical signs and weight loss of mice were constantly monitored from day 0 to 16 post-infection.

As shown in Figure 1, Mice infected with cold-passaged PT-IV-01 virus significantly reduced severity of clinical signs associated with influenza virus infection (Fig 1a). The mother strain infected group showed significant clinical signs as compared to the placebo control or PT-IV-01 virus inoculated groups (Fig. 1b). Clinical signs and weight loss was most prominent at 4 to 6 days post-infection although no mortality was observed with the mother strain. In contrast, the group of mice receiving PT-IV-01 virus did not show any clinical sign even at highest titer tested. These results suggested that the PT-IV-01 virus is highly attenuated. As a control, the virulence of A/PR/8/34 virus was also examined (Fig 1c). All mice succumbed after 5x10⁴ pfu dose of infection. It should be noted that the mother strain used for the generation of PT-IV-01 was reassortant carrying six internal genes derived from A/PR/8/34 strain. And yet, no mortality was observed for the mother strain even at the highest titer tested although partial virulence (as scored by weight loss) was significant. This result suggests that, as compared to the original A/PR/8/34 virus (Fig 1c), the mother strain was already partially attenuated (Fig 1b) even before cold-adaptation, and PT-IV-01 was further attenuated by cold-adaptation (Fig 1a). Therefore, the highly attenuated phenotype of PT-IV-01 was ascribed to double layer of attenuation, initially by repeated passage of mother strain in embryonated eggs (host-range phenotype) followed by further passage at low temperature (cold-adapted phenotype).

### <Example 5> Sequence analysis of the PT-IV-01 virus genome.

The entire genome of the cold-passaged PT-IV-01 virus was analyzed. The full genome segments of PT-IV-01 were amplified by RT-PCR, and then the PCR products were excised from agarose gel and used as templates for sequencing. The primers and reaction composition for transcription and amplification are listed in Table 3-1 and 3-2.

**Table 3-1. Primer sets**

| | Sense (SEQ ID NOs) | Anti-sense (SEQ ID NOs) |
|---|---|---|
| PB2 | agcgaaagcaggtcaattata (SEQ ID NO: 7) | agtagaaacaaggtcgtt (SEQ ID NO: 8) |
| PB1 | agcgaaagcaggcaaaccat (SEQ ID NO: 9) | agtagaaacaaggcattt (SEQ ID NO: 10) |
| PA | agcgaaagcaggtactgat (SEQ ID NO: 11) | agtagaaacaaggtactt (SEQ ID NO: 12) |
| NP | agcaaaagcagggta (SEQ ID NO: 13) | agtagaaacaagggtatt (SEQ ID NO: 14) |
| M | agcgaaagcaggtagat (SEQ ID NO: 15) | agtagaaacaaggtagtt (SEQ ID NO: 16) |
| NS | agcaaaagcagggtgacaaa (SEQ ID NO: 17) | agtagaaacaagggtgtt (SEQ ID NO: 18) |

**Table 3-2. Composition of PCR tube**

| Components | Volume (final concentration) |
|---|---|
| 10 X PCR Buffer(GIBCO-BRL) | 5 *µℓ* (1 X) |
| 50nM Magnesium Sulfate | 2 *µℓ* (2 mM) |
| 10nM dNTPs | 1 *µℓ* (0.2 mM) |
| 10 pmol/ *µℓ* sense primer | 2 *µℓ* (0.4 pmol/µℓ) |
| 10 pmol/*µℓ* anti-sense primer | 2 *µℓ* (0.4 pmol/µℓ) |
| Template(RT product) | 1 *µℓ*/3 *µℓ* (PA only) |
| Platinum Taq polymerase(GIBCO-BRL, 5unit/*µℓ*) or ExTaq(Takara, 5unit/ *µℓ*) | 0.2 *µℓ*(1unit) |
| Double distilled water | 36.8 *µℓ*/ 34.8 *µℓ* (PA only) |
| Total volume | 50 *µℓ* |

The RT reaction mixture was incubated at 37°C for 60min to induce cDNA synthesis, and then reaction tube was incubated at 94°C for 5min to inactivate reverse transcriptase. PCR was then proceeded as follows: initial denaturation at 94°C for 2min, followed by 40 cycles of 94°C for 30sec, 40-45°C for 30sec, and 68°C for 2min 30sec. A final incubation was performed at 68°C for 5min. At least four different internal primers were used for sequence analysis of each segment of PT-IV-01. When necessary, additional sequencing was performed for both DNA strands to confirm the sequence. The whole sequence of the six internal RNA genomes (PB1, PB2, PA, NP, M and NS) are shown in Sequence Listing (SEQ ID NOs: 1 to 6).

### <Example 6> Generation and characterization of reassortant virus

Live attenuated influenza vaccines are prepared by annual reassortment of the attenuated donor strain and contemporary epidemic strains. The segmented nature of RNA genome of influenza viruses allow reassortment of RNA segment to occur in doubly infected cells. Therefore, attenuated reassortant virus can be produced by mating an attenuated influenza virus strain PT-IV-01, which serves as a donor of attenuating genes, with a wild-type influenza virus. The resulting live attenuated reassortant virus (PT-IV-01 re) inherits the two RNA segments encoding hemagglutinin (HA) and neuraminidase (NA) surface glycoproteins from the contemporary epidemic virus and the six internal RNA segment from PT-IV-01.

The present disclosure generates reassortant influenza virus between PT-IV-01 and A/New Caledonia/20/99(H1N1) viruses. PT-IV-01 virus was mixed with the virulent wild-type A/New Caledonia/20/99(H1N1) virus in a predetermined ratio and inoculated into 11-day-old embryonated specific pathogen free (SPF) chicken eggs. After one day, the allantoic fluid was harvested and inoculated into new eggs in the presence of antibodies specific to PT-IV-01, and then the eggs were incubated at low temperature to select only viruses carrying the cold-adaptation property of the donor virus as well as outer envelop proteins HA and NA from the wild type virus. Virus clones obtained by plaque isolation on primary chicken embryo kidney (PCEK) or Mardin-Darby canine kidney (MDCK) cells in the presence of the same antibodies were grown again in fertilized eggs for further propagation. The reassortant virus clones were screened initially by hemagglutination inhibition and neuraminidase inhibition assay, and then their genetic composition was analyzed by multiplex RT-PCR. The final clone was named as PT-IV-01 re (A/NC). Similarly, reassortant virus between PT-IV-01 and A/Panama/2007/99 (H3N2) was generated and the final clone was named as PT-IV-01 re (A/PA).

The growth properties of the reassortant virus were measured at various temperatures in MDCK cells. As shown in Table 4, reassortant virus showed both cold-adapted and temperature-sensitive phenotype as expected from the parental OT-IV-01.

**Table 4**

| Strain | Virus titer(log10 pfu/ml) | | | |
|---|---|---|---|---|
| | 25°C | 33°C | 39°C | |
| PT-IV-01 re (A/NC) | 6 | 6.4 | <2.8 | 20 |
| PT-IV-01 re (A/PA) | 5.2 | 5.9 | <2.8 | |

### <Example 7> Analysis of immunogenicity and protective efficacy of PT-IV-01 re (A/NC)

The reassortant virus (1X10⁸ PFU per ml allantoic fluid), was obtained by inoculation with SPF(specific pathogen free) chicken eggs and incubation for three days at the temperature of 30°C. Likewise, wild type virus A/New Caledonia/20/99(H1N1) (3X 10⁷ PFU per ml allantoic fluid), was obtained by inoculation of SPF eggs and incubation at 37C for three days.

BALB/c mice received either reassortant virus (PT-IV-01 re (A/NC) or wild type virus A/New Caledonia/20/99(H1N1) at indicated titer (1X10⁶, 1X10⁵ and 1X10⁴ PFU/mouse). PBS (phosphate buffered saline) control was included for comparison. The mortality and morbidity (in terms of weight loss) were monitored daily after challenge. As shown in Figure 2, the PT-IV-01 re (A/NC) retained attenuated phenotype consistent with highly attenuated phenotype observed with the parental PT-IV-01 virus..

Mice were bled from retro-obital venous plexus 19-day after vaccination with reassortant viruses and induced antibody titers were determined by HI assay. As shown in Table 5, virus specific serum antibodies were detected in the sera of mice inoculated with all dose and these antibody titers were increased depending on inoculation titer.

**Table 5**

| Groups | HI titer |
|---|---|
| PBS | <32 |
| A/NC (H1N1) reassortant (1X10⁶) | 1184±352 |
| A/NC (H1N1) reassortant (1X10⁵) | 380±139.25 |
| A/NC (H1N1) reassortant (1X10⁴) | 312±104 |

Three weeks after vaccination, mice were challenged with A/New Caledonia/20/99(H1N1) wild type virus (1 LD₅₀). As shown in figure 3, all group of mice vaccinated with reassortant virus were did not show notable clinical signs or weight loss and survived the challenge infection of wild-type virus. In contrast, wilt type infection led to notable weight loss and 50% mortality at high dose of infection (1x10⁶ pfu). The results show that the PT_IV-01 re vaccine provdes efficient protection against infection by virulent viruses.

### <Example 8> Extremely early protection from heterologous challenge.

To evaluate prophylactic property of PT-IV-01 virus (H3N2), mice were vaccinated prior to virulent virus challenge. A group of 5 to 6-week-old BALB/c mice received 1.0×10⁶ PFU of PT-IV-01 virus by intranasal route at one day intervals up to four days before lethal infection with A/New Caledonia/20/99 (A/NC/99, H1N1). One group of mice were vaccinated and challenged simultaneously (mixed infection) to test potential interference of vaccine virus on virulence. Different from traditional vaccine approach, our strategy aims protection against various strains of influenza. Thus we selected A/NC/99 (H3N2) for challenge virus rather than same subtype (H1N1) influenza A virus. The interference was determined by daily examination on body weight (Figure 4a) and survival (Figure 4b).

Prior vaccination with the PT-IV-01 virus 1-4 days before challenge resulted in dramatic reduction of weight loss and mortality associated with influenza infection. The mortality was greatly reduced in groups vaccinated at least 1 day before challenge. Survival was 87.5% for vaccination groups of -1 and -2 day whereas 100% survival was achieved for the -3 and -4 day groups. Even in mixed infection, 25% mice survived while all mouse succumbed in non-vaccinated group.

The protection was most outstanding for vaccines 4 days before challenge (-4d group). The morbidity was greatly reduced as compared to other groups and the body weight regained only after 3 days post challenge. Mice that were vaccinated 3 days before challenge (-3d group) also resulted in 100% survival but clinical symptoms were more pronounced than the -4d group. The difference suggests a certain time intervals between vaccination and challenge are required for efficient protection. The group of mice that was vaccinated only 1 day before challenge (-1d group) showed notable clinical symptoms resulting in about 30% of body weight loss. And yet, compared to non-vaccinated group (NC only group), the morbidity and mortality were greatly reduced. Even when mice were simultaneously infected with PT-IV-01 and A/NC/99 (mix group), marked reduction in virulence was observed. The fact that even a mixed infection reduced toxic effect of A/NC/99, where the total virus titer that was introduced into mouse were 3-fold higher than A/NC/99 only group, strongly suggests interference of virulence by vaccine virus. Considering morbidity and mortality, it is concluded that protection is better in the order of -4d >-3d >-2d >-1d >0d (mixed infection). Overall result of heterologous protection is similar to heterotypic protection (example 10 and Figure 5). In general, better protection is provided with increased intervals between vaccination and challenge. Moreover, the effect of genetic interference between vaccine and virulent virus peaks at -2d and continues to decrease on -3d and -4d (example 14 and Table 7). This means that better protection from heterologous challenge on -3d or -4d group than - 2d group is not due to genetic interference but rather to innate immune response elicited by PT-IV-01 (example 13 and Fig 9).

### <Example 9> Determination of vaccine dose for early protection.

Vaccination induces adaptive immune response comprising antigen specific antibody responses and cell-mediated immune response that provides efficient protection. As is the case with adaptive immune response, innate immune response and genetic interference could also be influenced by the vaccine dose. However different dose strategy may be used to induce early protection against various subtypes. To test this, we vaccinated mice with 1.0×10³, 1.0×10⁴, 1.0×10⁵ and 1.0×10⁶ PFU of PT-IV-01 at one day before challenge with heterologous H1N1 virus (50× 10⁵ PFU).

Administration of mice with PT-IV-01 even at highest dose (1.0×10⁷ PFU) failed to show any clinical symptoms due to highly attenuated phenotype. We, therefore, set maximum vaccine dose to 1.0×10⁶ PFU that is 10 fold lower than virus titer that could be used in our experimental setting without notable clinical signs. Mice vaccinated in 1.0×10³ PFU at 1 day before challenge was 100% lethal showing that 1.0×10³ PFU is insufficient titer to induce early protection. Another groups of mice were vaccinated with 1.0×10⁴, 1.0×10⁵, 1.0×10⁶ PFU and survived 25, 50, 87.5% respectively (Table 6). From these results, we conclude higher vaccine dose are required for better protection by vaccination at 1 day before challenge.

**Table 6. Survival rate of mice vaccinated at different doses**

| Group | Survival |
|---|---|
| No challenge | 8/8 (100%) |
| 1.0×10⁶ PFU | 7/8 (87.5%) |
| 1.0×10⁵ PFU | 4/8 (50%) |
| 1.0×10⁴ PFU | 2/8 (25%) |
| 1.0×10³ PFU | 0/8 (0%) |
| No vaccination | 0/10 (0%) |

### <Example 10> Extremely early protection from heterotypic challenge

The live vaccine was also evaluated for protection against lethal influenza B virus challenge. Experimental schedule is essentially same as shown in example 8 but different challenge virus B/Shangdong/7/97 was used. Lethal dose of B/Shangdong/7/97 is 1.5×10⁵ PFU and 2 LD₅₀ (1.5×10⁵ PFU) of virus were used for challenge mice.

Similar pattern of body weight loss were observed as in heterologous interference (Figure 5a). 100% survival and reduced morbidity was shown in mouse vaccinated -3 and -4 days before B/SD/97 challenge, while 75% survival was shown for the -1, -2 day and mix groups (Figure 5b).

Reassortants between influenza A and B viruses are not observed in nature nor readily generated in laboratory (J Virol 77, 8031-8038, 2003), and therefore, the heterotypic protection conferred by influenza A vaccine from lethal challenge by the influenza B virus is due to the innate immune response rather than to genetic interference between the vaccine (PT-IV-01) and the challenge virus (see example 14 and Table 7). Consistent with this interpretation is that there is a degree of specificity between influenza A and B strains in their recognition of promoters (J Virol 70, 1232-1236, 1996). The results further extend protection from heterologous challenge (example 8, Figure 4) that innate immune response plays a greater role than genetic interference for early protection.

### <Example 11> Extremely early protection from avian influenza infection

From the results described previously, we can expect immediate and broad-spectrum protection against various influenza strains by administration of donor virus. We further examined the early protection from H5 subtype avian influenza virus which is closely related to potential pandemic candidate. Experiment was proceeded as explained in example 8. We vaccinated mice with PT-IV-01 1 and 4 days before challenge (Figure 6).

Vaccination with PT-IV-01 1day or 4day prior challenge with A/Aquatic Bird/Korea/W81/06 (H5N2) significantly reduced clinical symptoms associated with virulent infection. Mice vaccinated at 4 days before challenge exhibited more efficient protection than the groups immunized 1 day before.

### <Example 12> Early protection is provided without specific antibody responses

Generally, vaccination with influenza vaccine stimulates adapted immune responses and generates antigen specific antibody production. These antibodies are key mediator in clearance of followed virus infection. However, the fact that early interference exhibited similar protection to various strains may suggest neutralizing acquired immune responses are not heavily involved. To prove this possibility, we examined antibody responses at early time points after vaccination in mice.

PT-IV-01 virus specific IgA antibody in BAL fluid (Figure 7a) and IgG antibody in serum (Figure 7b) of vaccinated or PBS controlled mice were tested for their specificity to virus by enzyme-linked immunosorbent assay (ELISA).

A microwell plate was coated with 5.0×10⁵ PFU of PT-IV-01 virus in PBS buffer (pH 7.4). After washing with 50mM Tris-HCl containing 0.05% Tween-20, plate was blocked with PBS containing 1% BSA for 1 hour at room temperature. BAL fluid and serum samples were added to each well and incubated for 1 hour at room temperature. After washing, HRP conjugated anti-mouse IgG/IgA monoclonal antibody was added and incubated for 1 hour. After washing, tetramethylbenzidine (TMB) solution was added, incubated for 30 minutes at room temperature, and stop solution (2N H₂SO₄) was added. The absorbance was measured at 450nm on ELISA reader. Antibody titers were checked for 7 days and elevation of IgA and IgG level was observed at least 5 days post vaccination. As evident in Figure 7, there was no detectable production of virus specific antibody until 5 days post vaccination either in IgA in BAL fluid (Figure 7a) or in IgG in serum (Figure 7b).

Influenza B virus infection was effectively prevented by vaccination with influenza A vaccine strain PT-IV-01 (example 10). We therefore tested if PT-IV-01 specific antibody can interact with influenza B virus. We used ELISA as described earlier using microwells coated with either A/New Caledonia/20/99 or B/Shangdong/7/97 (Figure 8).

PT-IV-01 virus specific IgG antibodies interacted with A/NC/99, whereas cross-reactivity with B/SD/97 was hardly observed (Figure 8). Furthermore, novel protection of PT-IV-01 against influenza B virus without antibody cross-reactivity (example 10, Figure 5) strongly implies the involvement of broad-spectrum innate immune response.

### <Example 13> Analysis of pro-inflammatory cytokines stimulated by PT-IV-01

Innate immune response provides first line of defense from viral infection. Different from acquired immune response which induces selective protection, innate immune response provides resistance to various pathogens in a short period. We examined whether innate immune response by vaccination with donor virus contributes to early interference.

We used IL-6, TNF-alpha, IL-1 beta for marker cytokines for innate immune response. Specific antibodies against these pro-inflammatory cytokines are used in ELISA to analyze cytokine level of BAL fluid samples obtained from mice vaccinated with PT-IV-01 virus. Groups of mice were vaccinated with 1.0×10⁶ PFU of donor virus and BAL fluid were prepared for 4 days. In comparison, the cytokine level was also monitored from mice infected with the same titer (1.0×10⁶ PFU) of the mother strain before cold-adaptation (Figure 9).

PT-IV-01 virus infection stimulated lower, but distinct level of pro-inflammatory cytokines when compared to the mother strain infection. These results may reflect attenuated phenotype of PT-IV-01 virus. The decrease of pro-inflammatory cytokines was most pronounced on the level of TNF-α among all cytokines monitored in our experiment. And yet, the level of IL-6 and IL-1β is still maintained. IL-1β was stimulated at a low level in both the mother strain and PT-IV-01 infected mice. From these results, we conclude that vaccination with the cold-adapted PT-IV-01 stimulates milder but distinct innate immunity than strong and acute inflammation frequently associated with virulent infection.

### <Example 14> Genetic interference plays a minor role in early protection

In a mixed infection of A/NC/99 and PT-IV-01, the survival rate was markedly improved as compared with A/NC/99 only infection (Figure 1 and 2). Under this condition, the innate immune response is not expected to play significant role on protection. We therefore considered possibility of interference in gene level.

The PT-IV-01 virus used in the present study has accumulated 16 nucleotide substitutions in all 6 internal genomes during subsequent passage at low temperature, which collectively contributes to attenuated phenotypes.

To estimate direct interference among influenza RNAs, several groups of mice were vaccinated and challenged at various time intervals as in example 8. After 24 hours post challenge, viruses were isolated from infected mice, plaque purified, and the RNA composition was analyzed by the multiplex RT-PCR method. For the detection of PT-IV-01 derived RNAs, we used 8 pairs of PT-IV-01 specific primers, which has previously been confirmed for their specificity.

50 independent plaques were isolated from lung homogenate of the infected mice for each group and total 250 different plaque isolates were analyzed (table 7). We observed significant population of reassortants (10-30%) among different groups of varying intervals of vaccination and challenge, where the reassortant population was highest (46%) in the mixed infection. Generation of reassortant shows that multiple infections occurred in the epithelial cells of mouse respiratory tract and suggests the possibility of competition between the two viral species in gene level.

Example 10 (and Figure 5) showed that protection is better in the order of -4d >-3d >-2d >-1d > 0d (mixed infection). Table 7 actually shows that the genetic interference between vaccine and virulent virus actually decreases on -3d and -4d group. The results shows that the genetic interference plays minor role, if at all, to early protection whereas broad-spectrum innate immune response plays a major role.

**Table 7. Ratio of reassortant virus generated by vaccine strain PT-IV-01 and the virulent A/NC/99**

| Group | n*^{a}* | Viruses analyzed by multiplex RT-PCR | | | Ratio of reassortant |
|---|---|---|---|---|---|
| | | PT-IV-01 | A/NC/99 | Reassortant | |
| -4d | 50 | 0 | 45 | 5 | 10% |
| -3d | 50 | 4 | 36 | 10 | 20% |
| -2d | 50 | 27 | 8 | 15 | 30% |
| -1d | 50 | 11 | 33 | 6 | 23% |
| mix | 50 | 6 | 21 | 23 | 46% |
| Total | 250 | 48 | 143 | 59 | 24% |

| | | | | | |
|---|---|---|---|---|---|
| *^{a}* Number of tested plaques | | | | | |

### <Example 15> Analysis of immunogenicity of PT-1V-01 based reassortant vaccine (for illustrative purposes only)

Mice were anaesthetized with pentobarbital sodium (55 mg/kg/0.2 ml) by intra-peritoneal (i.p.) route, and vaccinated with 50 ul of the PT-IV-01 re (A/NC) or PT-IV-01 re(A/PA) by intranasal (i.n.) route. And negative control mice were infected with the same volume of buffer used for vaccine formulation. The mice were sacrificed 4 weeks later, and the sera, nasal wash (mucosal fluid) and BAL (Bronchoalveolar lavage, lung airway) fluid were collected and analyzed by HI and EIA. To collect BAL fluid, a 1-mL syringe (26G needle) containing 1 mL of PBS was inserted into the trachea to the lung direction and tightened with a string to prevent the leakage of solution. Pumping of the PBS was repeated 3 times. Collected fluid was transferred to a new tube containing 10 ul of 100 x protease inhibitor cocktail (PIC, 100 µM Pepstatin A, 1 mM Leupeptin, 20 mM Pefabloc SC (AEBSF)). The solution was centrifuged at 10,000 rpm for 5 min, and then 0.5 mL of the supernatant was transferred to a new tube containing 5 µL of 10 x PIC and 50 µL of PBS (0.1% BSA). BAL fluid was stored at -20°C until use.

After the collection of BAL fluid, the under jaw of the mouse was removed, and a 1-mL syringe containing 1 mL of PBS (0.1% BSA) was inserted into naso-pharynx. While clipping with forceps prevented the back-flow, nose was washed. Collected fluid, nasal wash was transferred to a new tube containing 10 µL of 100 x protease inhibitor cocktail (PIC, 100 µM Pepstatin A, 1 mM Leupeptin, 20 mM Pefabloc SC (AEBSF)). The solution was centrifuged at 10,000 rpm for 5 min, and then 0.5 mL of the supernatant was transferred to a new tube containing 5 µL of 10 x PIC and 50 µL of PBS (0.1% BSA). Nasal wash was stored at -20°C until use.

The amount of antibody was assayed by HI and EIA. The HI titer was taken as the highest dilution factor that gave inhibition of hemagglutination against eight HA units of virus, and the titer of EIA was the highest dilution factor which gave absorbance above 0.2. EIA titers were calculated according to the method of Reed and Muench (Vaccine 21, 940- 945, 2003).

### <Example 16> Determination of MID₅₀ (Fifty percent mouse infectious dose) (for illustrative purposes only)

Balb/c mice (5-week-old female of 16~20 g, Biogenomics co., Korea) were used to determine MID₅₀ titer of wt viruses. The animals were fed in isolated room (20 - 26°C, 40 - 60% humidity, 150 ~ 300 Lux, 12 h illumination) until use. Allantoic fluid containing virus was 10-fold serially diluted with NAF/SPG (Normal allantoic fluid/SPG).

Mice were anaesthetized with pentobarbital sodium (55 mg/kg/0.2 ml) by intraperitoneal (i.p.) route, and infected with 50 µL of the diluted virus solution by intranasal (i.n.) route. Control mice were infected with the same volume of NAF/SPG. The mice were sacrificed 3 weeks later, and their sera were collected and analyzed by HI. Since the protective level of HI is 40, the blood samples showing HI higher than 40 were considered as positive sera. MID₅₀ titers were calculated according to the method of Reed and Muench.

### <Example 17> Protective efficacy of PT-IV-01 re(A/NC) against virulent challenge (for illustrative purposes only)

Four weeks after immunization with PT-IV-01 reassortant vaccine, mice were anaesthetized with pentobarbital sodium (55 mg/kg/0.2 ml) by intraperitoneal (i.p.) route, and challenged with 50 ul containing 100 MID₅₀ (Fifty percent mouse infectious dose) of virus by i.n. route. Control mice received the same volume of NAF/SPG. The mice were sacrificed 4 days later, and their lungs and nasal wash were collected. The lungs were homogenized in 1 ml of cold PBS including antibiotics to prevent microbial growth and the homogenates were frozen at -70°C and later thawed and pelleted by centrifugation. Virus content was analyzed by plaque assay and real-time PCR.

The data are summarized in Fig. 10. Vaccination with PT-IV-01 re(A/NC) induced IgG effectively whereas 0.75 ug HA of inactivated vaccine per mouse did not induce enough level of IgG. And secretory IgA (sIgA) was detected in BAL and nasal wash of mouse vaccinated with PT-IV-01 re(A/NC) but not with inactivated vaccine. The vaccine dose of 8.9×10² PFU per mouse (group 2) was enough to completely clear the virulent A/NC/20/99 from the lung of infected mouse even without high level of antibodies. HI titer of sera from mice vaccinated with inactivated vaccine (group 3) was higher than the titer of group 2, and yet failed to clear viruses completely. This means that sIgA induced by PT-IV-01 re(A/NC) neutralize challenge viruses and protect mice more effectively. The data demonstrate that PT-IV-01 based reassortant vaccine confers much better protection as compare to conventional inactivated vaccine.

### <Example 18> Protective efficacy of PT-IV-01 re(A/PA) against virulent challenge (for illustrative purposes only)

Four weeks after immunization with PT-IV-01 re(A/PA), mice were anaesthetized with pentobarbital sodium by intraperitoneal (i.p.) route, and challenged with 50 ul containing 100 MID₅₀ (Fifty percent mouse infectious dose) of A/Panama/2007/99 by i.n. route. Control mice received the same volume of NAF/SPG. The mice were sacrificed 4 days later, and their lungs and nasal wash were collected. The lungs were homogenized in 1 ml of cold PBS including antibiotics to prevent microbial growth and the homogenates were frozen at -70°C and later thawed and pelleted by centrifugation. Virus content was analyzed by plaque assay and real-time PCR.

As shown in Fig. 11, accination with PT-IV-01 re(A/PA) induced IgG effectively and cleared the infecting virus form the lung completely. The secretion of sIgA dependes on experimental conditions, and sIgA was evident with vaccination dose of 1 x 10^{5.5} TCID₅₀. The vaccine dose of 1.9x10⁵ PFU per mouse (group 1) was enough to completely clear the virulent A/PA/2007/99 from the lung of infected mouse. Even hundred-fold lower dose of vaccination (1.9x10³ PFU) was enough to lower the infectous virulent virus by 20 fold as compared to without vaccination (Group 4). The data again demonstrate that PT-IV-01 bases reassortant vaccine confers an extreme good protection from lethal challenge. The results were confirmed in a separate and independent set of experiment (Fig. 12).

### <Example 19> Protective efficacy of trivalent vaccine containing A/H1N1, A/H3N2 and B virus components (for illustrative purposes only)

We blended three reassortant viruses, PT-IV-01 re(A/NC)(H1N1), PT-IV-01 re(A/PA) (H3N2) and B/SD reassortant as live trivalent vaccine. Mice were immunized with the trivalent vaccine by i.n. route. Monovalent vaccine PT-IV-01 re(A/NC)(H1N1) was included as control. The immunogenicity and protective efficacy from virulent challenge were compared with each other (Fig 13). In trivalent vaccine formulation (group 2), the level of antibody response was slightly lower than that of monovalent virus vaccine (group 1), and yet was enough to completely clear the virulent virus from infected lung. The protective efficacy of inactivated vaccine was only marginal in trivalent vaccine formula (group 3) and was not enough to clear the virulent virus form the lung as compared to the control without vaccination (group 4). The result shows that PT-IV-01 reassortant vaccine exhibits effective protection in trivalent vaccine formula as well as in monovalent formulation. The results also suggest that multi-valent vaccine formulation could be used for cross-protection against variety of subtypes of infleunza viruses.

### INDUSTRIAL APPLICABILITY

The present invention relates to a live vaccine comprising an attenuated influenza virus strain. The attenuated influenza virus strain and the live vaccine of the present disclosure are useful for prevention of seasonal influenza episodes and sudden outbreak of influenza pandemics of predicted or unknown identity, since they have safety, efficacy, high production yield, immediate protection against variety of influenza subtypes and prolonged protection against specific influenza subtype.

## Claims

1. A live vaccine comprising an attenuated influenza virus strain as an effective ingredient and at least one pharmaceutically acceptable carrier or excipient for use in a method for inducing early protection against a human influenza virus or an avian influenza virus, wherein the early protection is protection of the host from influenza infection at one day from vaccination, and wherein the attenuated influenza virus strain is preparable by cold adaptation of a mother strain which carries 6 internal genome segments of A/PR/8/34(H1N1) and two surface antigens hemagglutinin (HA) and neuramidase (NA) of A/Aichi/2/68(H3N2) and the attenuated influenza virus strain comprises RNA genome segments having nucleotide sequences corresponding to the DNA sequences of SEQ ID NOs: 1 to 6.

## Patentansprüche

1. Lebendimpfstoff, umfassend einen abgeschwächten Influenzavirusstamm als wirksamen Inhaltsstoff und mindestens einen pharmazeutisch geeigneten Träger oder Exzipienten für die Verwendung in einem Verfahren zum Induzieren eines frühen Schutzes gegen ein humanes Influenzavirus oder ein aviäres Influenzavirus, wobei es sich bei dem frühen Schutz um den Schutz des Wirts vor einer Influenzainfektion ab einem Tag nach der Impfung handelt und wobei der abgeschwächte Influenzavirusstamm durch Kälteadaption eines Mutterstammes hergestellt werden kann, der 6 interne Genomsegmente von A/PR/8/34(H1N1) und zwei Oberflächenantigene Hämagglutinin (HA) und Neuraminidase (NA) von A/Aichi/2/68(H3N2) trägt, und der abgeschwächte Influenzavirusstamm RNA-Genomsegmente enthält, die Nukleotidsequenzen aufweisen, welche den DNA-Sequenzen von SEQ ID NR. 1 bis 6 entsprechen.

## Revendications

1. Vaccin vivant comprenant une souche atténuée du virus de la grippe comme composante efficace et au moins un support ou excipient pharmaceutiquement acceptable destiné à l'utilisation dans un procédé pour induire une protection rapide contre un virus de la grippe humaine ou un virus de la grippe aviaire, dans lequel ladite protection rapide est une protection de l'hôte contre la grippe un jour après la vaccination, et dans lequel la souche atténuée du virus de la grippe est préparée par une adaptation au froid d'une souche mère porteuse de 6 segments de génome internes de A/PR/8/34(H1N1) et deux antigènes de surface hémagglutinine (HA) et neuraminidase (NA) de A/Aichi/2/68(H3N2)et la souche atténue du virus de la grippe comprend des segments de génome ARN ayant des séquences de nucléotides correspondant aux séquences ADN de SEQ ID NO: 1 à 6.
